# EUROPEAN PATENT SPECIFICATION

(11) **EP 2 231 860 B1**
(45) Date of publication and mention of the grant of the patent: **05.10.2011**
(21) Application number: 08862011.7
(22) Date of filing: 17.12.2008
(51) Int. Cl.: C12N 15/10, C07K 1/04, G01N 33/68, C07K 14/31, C07K 14/49

(54) **POLYPEPTIDE DERIVED FROM PROTEIN A AND ABLE TO BIND PDGF**
PDGF-BINDENDES POLYPEPTID AUS PROTEIN A
POLYPEPTIDE DÉRIVÉ D'UNE PROTÉINE ET SE LIANT AU PDGF

(30) Priority: 19.12.2007 US 2972; 21.12.2007 EP 07150394; 26.12.2007 US 9171 P
(43) Date of publication of application: 29.09.2010
(73) Proprietor: Affibody AB, 161 02 Bromma (SE)
(72) Inventor: LINDBORG, Malin, S-126 53 Hägersten (SE); GUNNERIUSSON, Elin, S-133 36 Saltsjöbaden (SE); LENDEL, Christofer, S-123 71 Farsta (SE)
(74) Representative: Mattsson, Niklas
(86) International application number: PCT/EP2008/010776
(87) International publication number: WO 2009/077175

(56) References cited:
- WO-A-91/01743
- WO-A-95/19734

## Description

### Field of the invention

The present invention relates to polypeptides that bind to platelet-derived growth factor receptor beta (PDGF-Rβ). It also relates to new methods and uses of such polypeptides in treatment and diagnosis of different PDGF-Rβ related conditions.

### Background

The platelet-derived growth factor receptor beta (PDGF-Rβ) is a membrane-spanning tyrosine kinase. The ligand PDGF is composed of combinations of the homologous chains A, B, C and D, combined to either homo- or heterodimers. PDGF-Rβ binds PDGF-BB with high affinity and PDGF-AB with lower affinity. Ligand binding leads to dimerization and trans-phosphorylation of tyrosines in the intracellular kinase domain of the receptors.

PDGF is an important factor for regulating cell proliferation, cellular differentiation, cell growth and development. PDGF-Rβ is implicated in angiogenesis and in early stages of fibrosis. This receptor represents an attractive and potentially valuable target, e.g. for treatment and molecular imaging in for example oncologic and fibrotic diseases.

Antibodies blocking the effect of PDGF-Rβ are in clinical development and the continued provision of agents with a comparable affinity for this receptor remains a matter of substantial interest within the field. Of great interest is also the provision of uses of such molecules in the treatment and diagnosis of disease. It is an object of the invention to provide new PDGF-Rβ-binding agents, which could for example be used for diagnostic, *in vitro* or *in vivo* imaging, and therapeutic applications.

### Summary of the invention

According to one aspect of the invention, there is provided a platelet derived growth factor receptor beta (PDGF-Rβ) binding polypeptide, comprising a platelet derived growth factor receptor beta binding motif, *PBM*, which motif consists of an amino acid sequence selected from
i) EX₂X₃X₄AAX₇EID X₁₁LPNLX₁₆X₁₇X₁₈QW NAFIX₂₅X₂₆LX₂₈X₂₉,
   wherein, independently of each other,
   X₂ is selected from L, R and I;
   X₃ is selected from R, I, L, V, K, Q, S, H, and A;
   X₄ is selected from A, R, N, D, Q, E, H, K, M, S, T, W, F and V;
   X₇ is selected from A, R, D, Q, E, G, K and S;
   X₁₁ is selected from A, R, N, D, E, G, K, S, T and Q;
   X₁₆ is selected from N and T;
   X₁₇ is selected from R and K;
   X₁₈ is selected from A, R, N, D, C, Q, E, G, L, K, M, S, T, W and V;
   X₂₅ is selected from K, R, Q, H, S, G and A;
   X₂₆ is selected from S and K;
   X₂₈ is selected from V, R, I, L and A;
   X₂₉ is selected from D and K; and
ii) an amino acid sequence which has at least 90 % identity to the sequence defined in i),
   and wherein the PDGF-Rβ-binding polypeptide binds to PDGF-Rβ such that the K_{D} value of the interaction is at most 1 x 10⁻⁶ M.

The above definition of a class of sequence related, PDGF-Rβ-binding polypeptides according to the invention is based on an analysis of a number of random polypeptide variants of a parent scaffold, that were selected for their interaction with PDGF-Rβ in selection experiments. The identified PDGF-Rβ-binding motif, or "*PBM*", corresponds to the target binding region of the parent scaffold, which region constitutes two alpha helices within a three-helical bundle protein domain. In the parent scaffold, the varied amino acid residues of the two *PBM* helices constitute a binding surface for interaction with the constant Fc part of antibodies. In the present invention, the random variation of binding surface residues and the subsequent selection of variants have replaced the Fc interaction capacity with a capacity for interaction with PDGF-Rβ.

As the skilled person will realize, the function of any polypeptide, such as the PDGF-Rβ-binding capacity of the polypeptides according to the invention, is dependent on the tertiary structure of the polypeptide. It is therefore possible to make minor changes to the sequence of amino acids in a polypeptide without affecting the function thereof. Thus, the invention encompasses modified variants of the *PBM* of i), which are such that the resulting sequence is at least 90 % identical to a sequence belonging to the class defined by i), such as at least 93 % identical, such as at least 97 % identical. For example, it is possible that an amino acid residue belonging to a certain functional grouping of amino acid residues (e.g. hydrophobic, hydrophilic, polar etc) could be exchanged for another amino acid residue from the same functional group.

In one embodiment of the polypeptide according to the invention, X₁₇ is R.

In one embodiment of the polypeptide according to the invention, X₁₁ is selected from A, R, N, D, E, G, K, S and T.

In one embodiment of the polypeptide according to the invention, X₂ is selected from L and R.

In one embodiment of the polypeptide according to the invention, X₃ is selected from R, I, L, V and S.

In one embodiment of the polypeptide according to the invention, X₄ is selected from A, R, N, D, Q, E, H, K, M, S, T and W.

In one embodiment of the polypeptide according to the invention, X₂₅ is selected from K, R, Q, H and S.

In one embodiment of the polypeptide according to the invention, X₂₈ is selected from V, R, I and L.

In one embodiment of the polypeptide according to the invention, X₂₉ is D.

In one embodiment of the polypeptide according to the invention, X₂₅ is K.

In one embodiment of the polypeptide according to the invention, X₂₈ is V.

In one embodiment of the polypeptide according to the invention, X₃ is selected from I and V.

In one embodiment of the polypeptide according to the invention, X₄ is selected from A, R, E and K.

In one embodiment of the polypeptide according to the invention, X₇ is selected from A, R and E.

In one embodiment of the polypeptide according to the invention, X₁₁ is selected from A, R, N and E.

In one embodiment of the polypeptide according to the invention, X₁₈ is selected from R, E, K and V.

In one embodiment of the polypeptide according to the invention, X₂ is L.

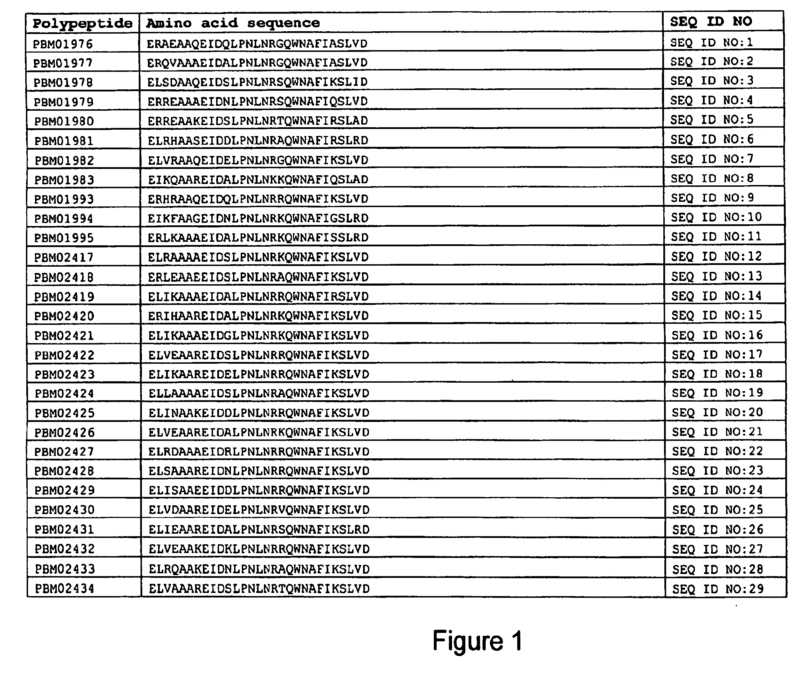

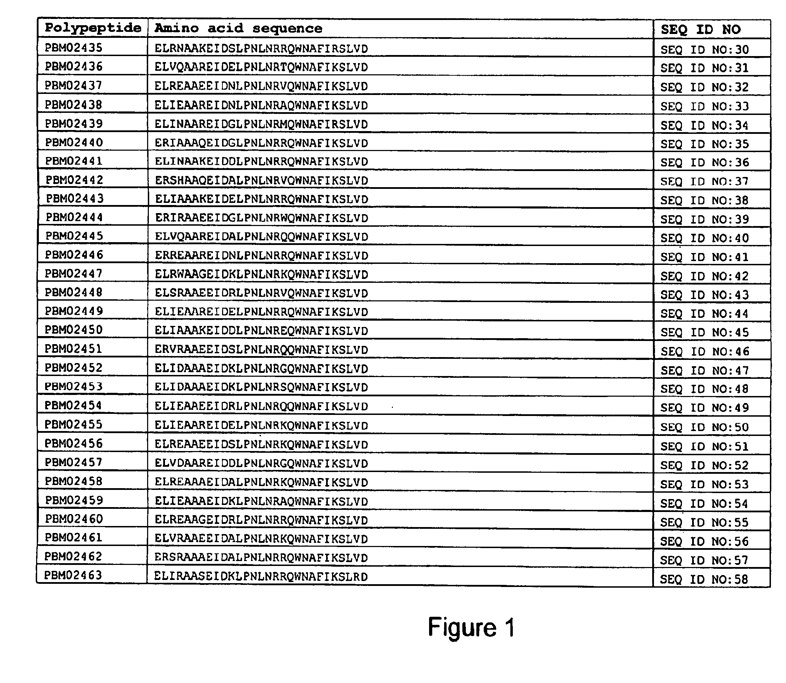

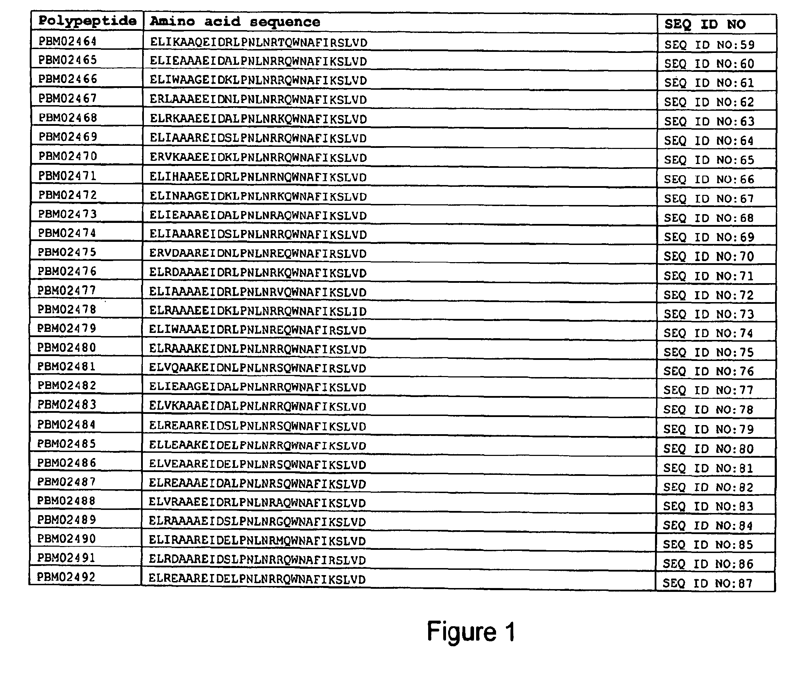

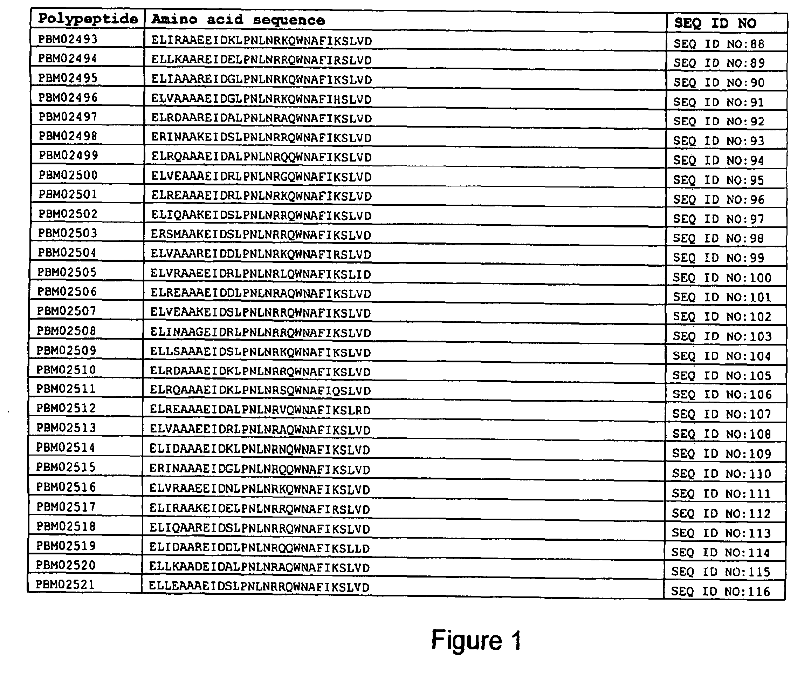

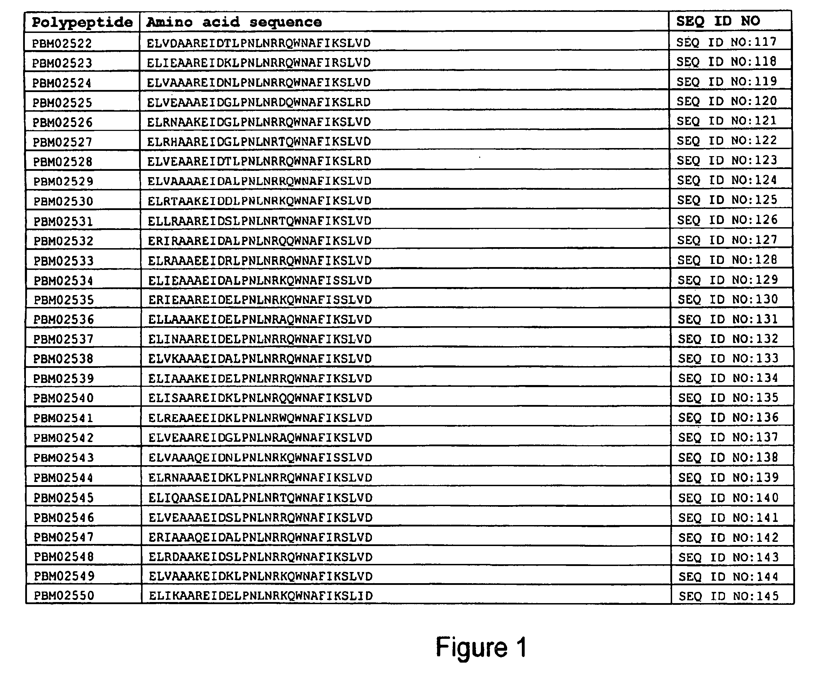

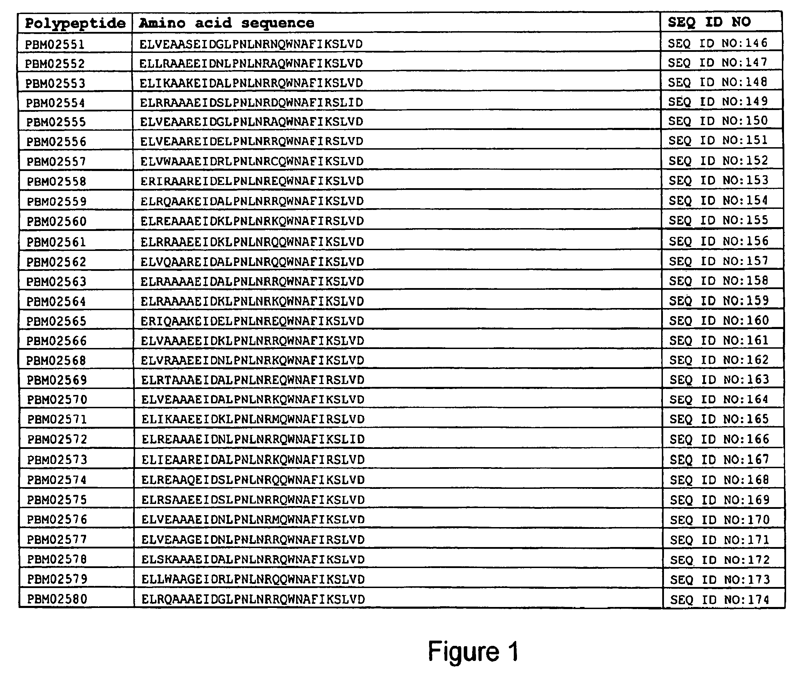

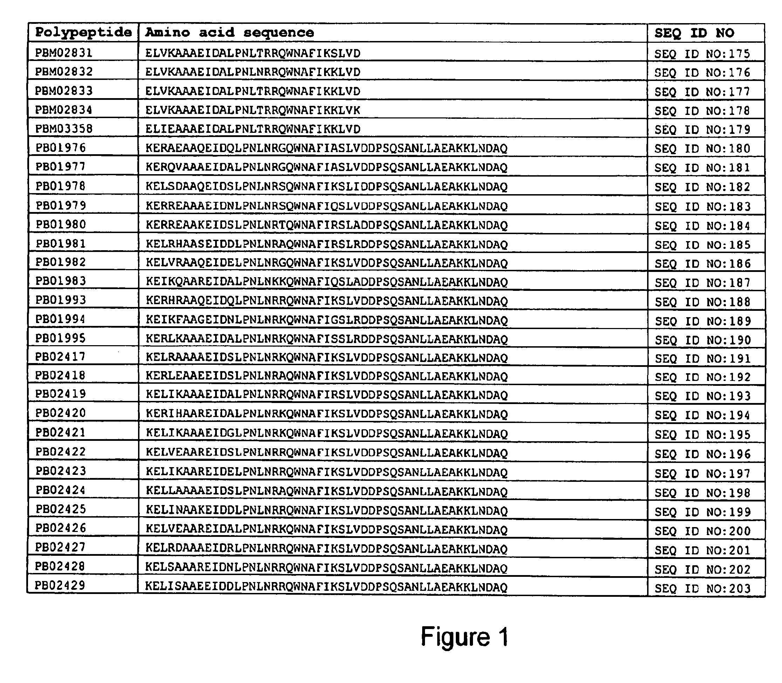

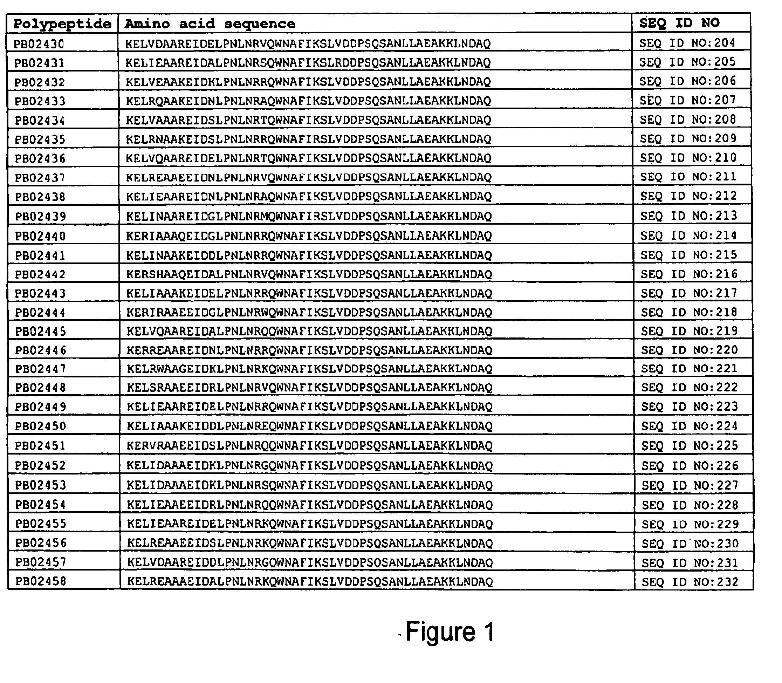

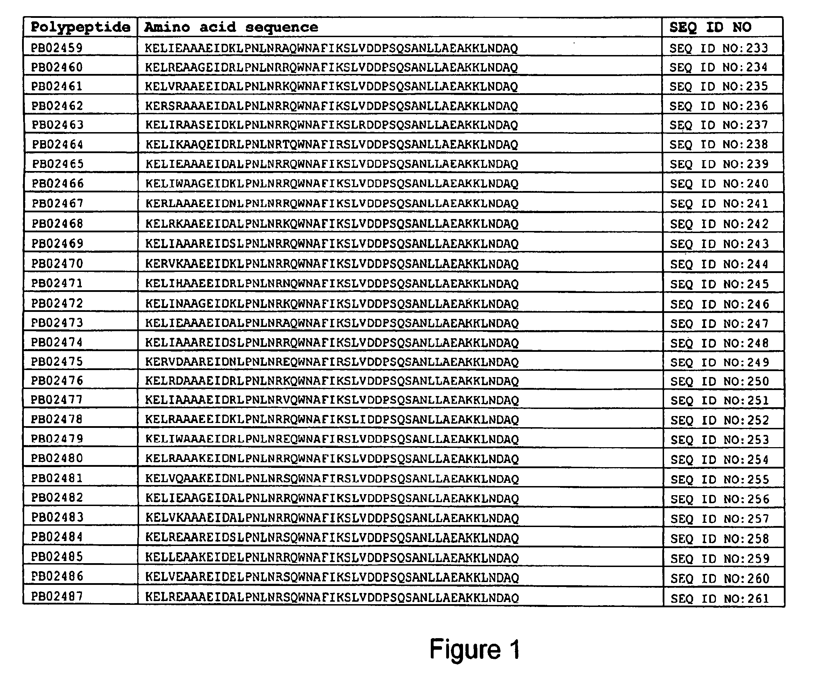

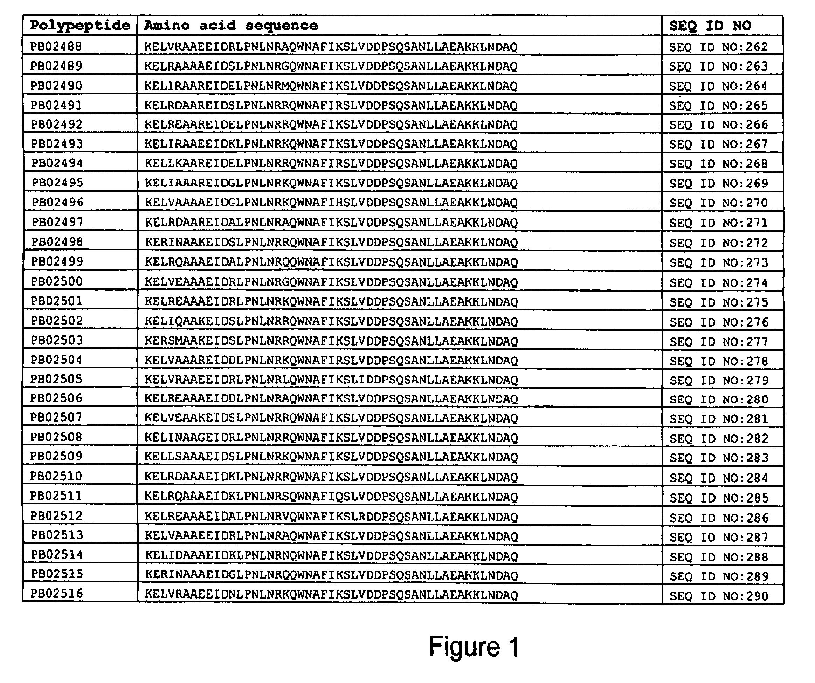

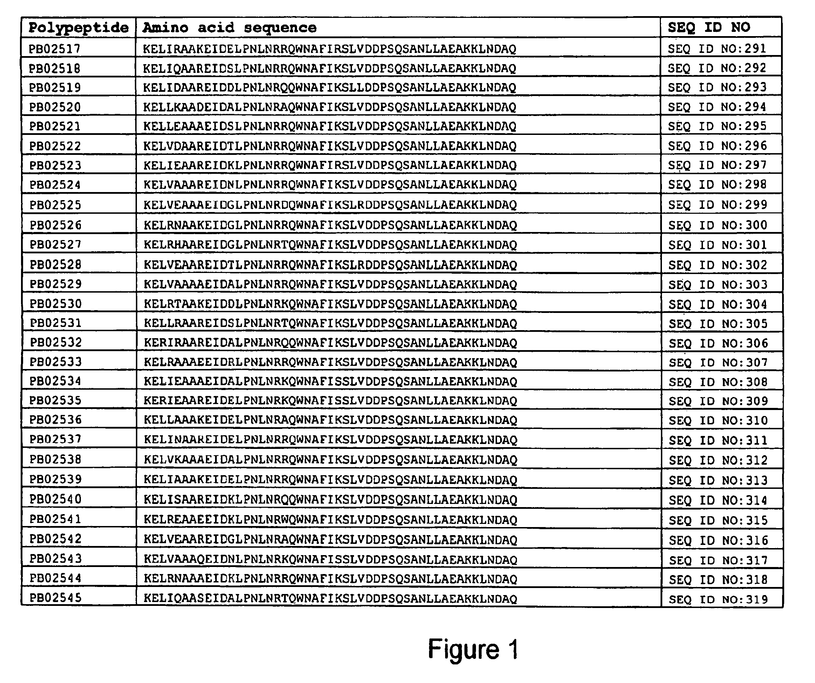

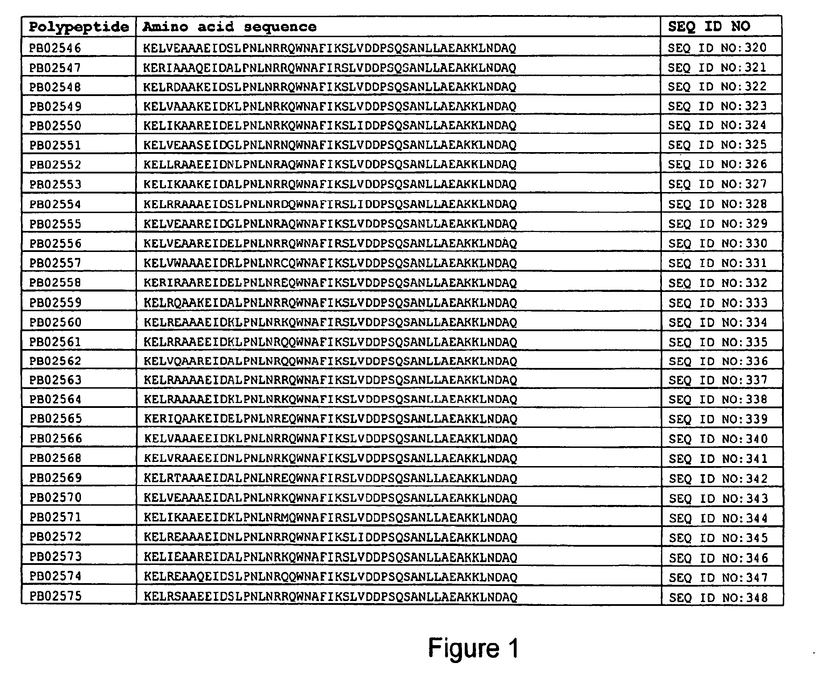

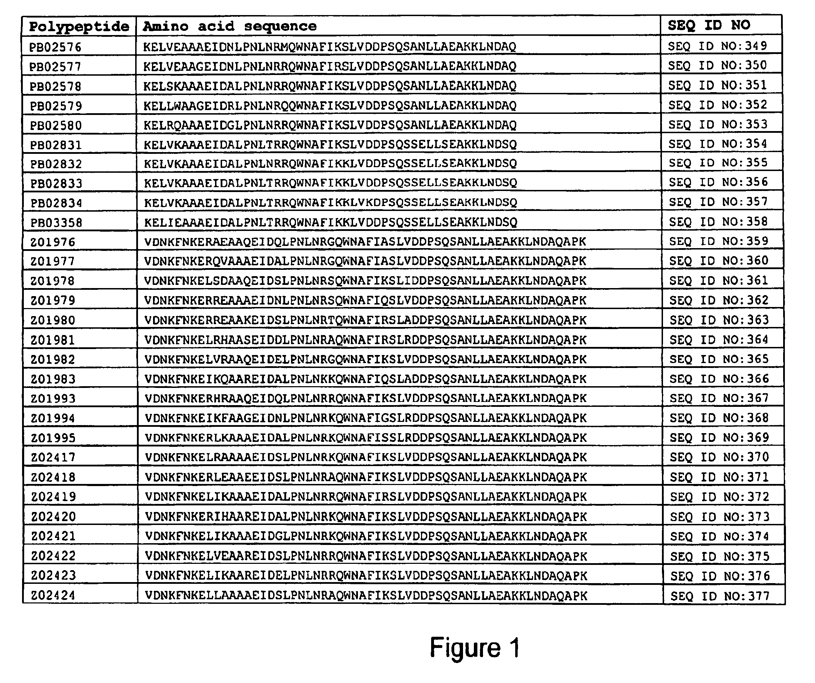

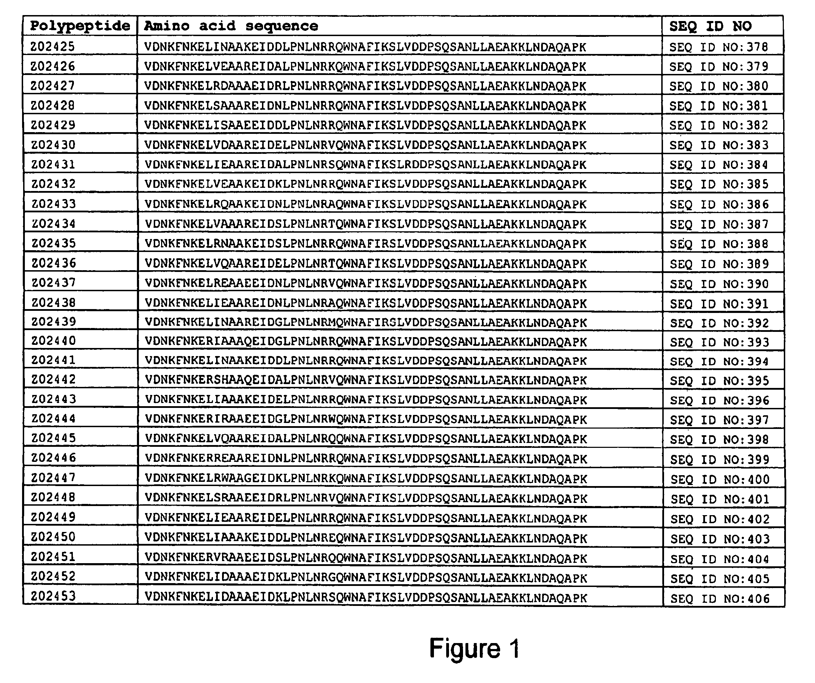

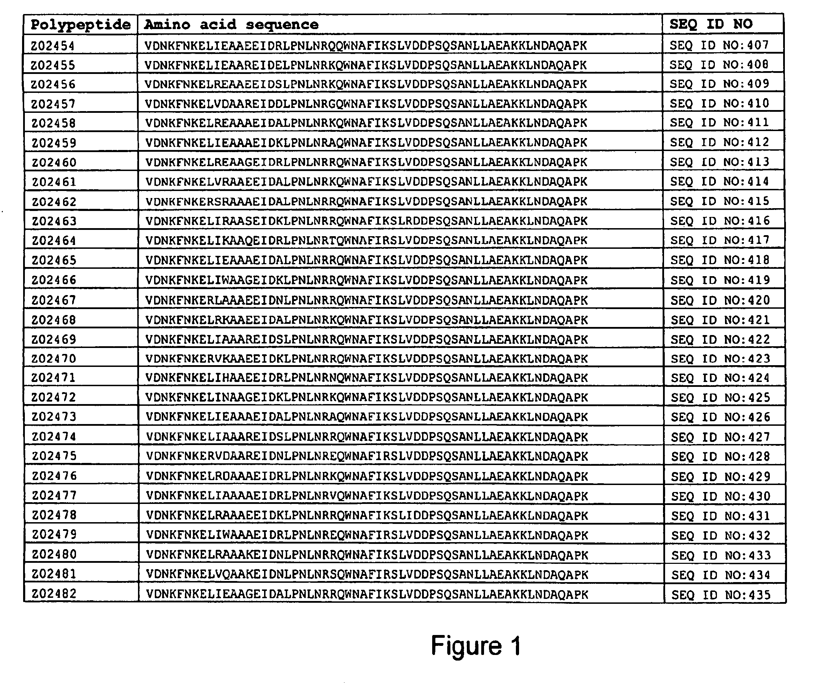

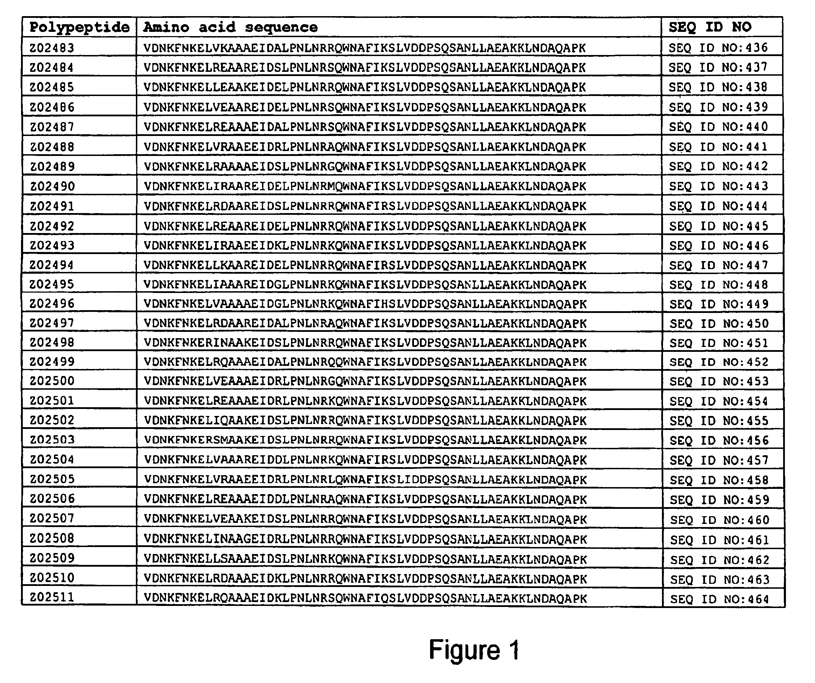

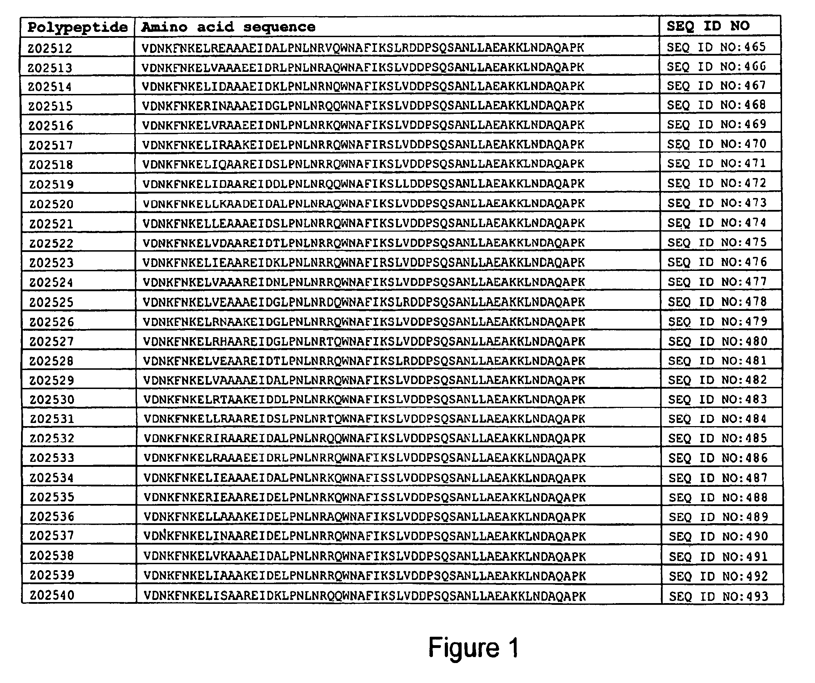

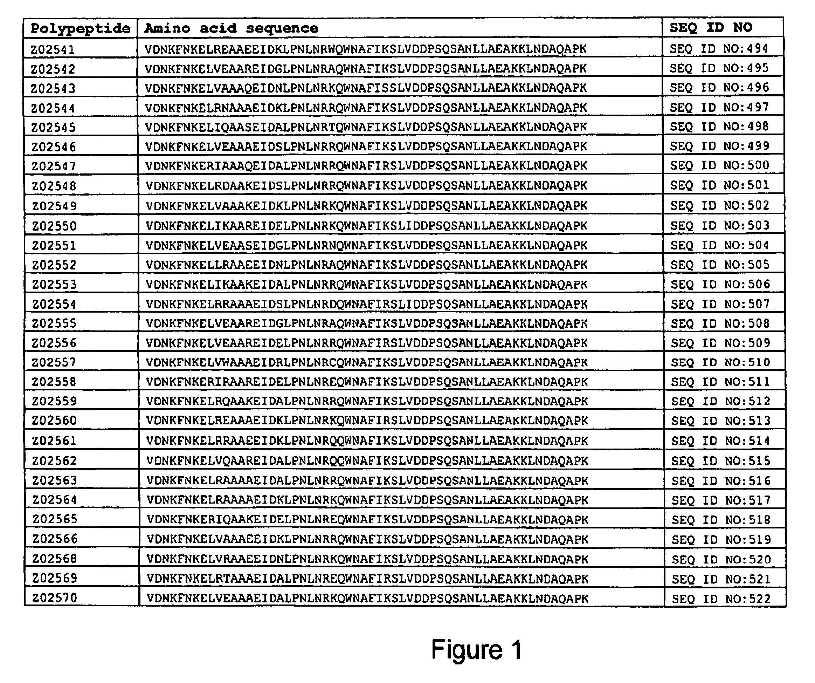

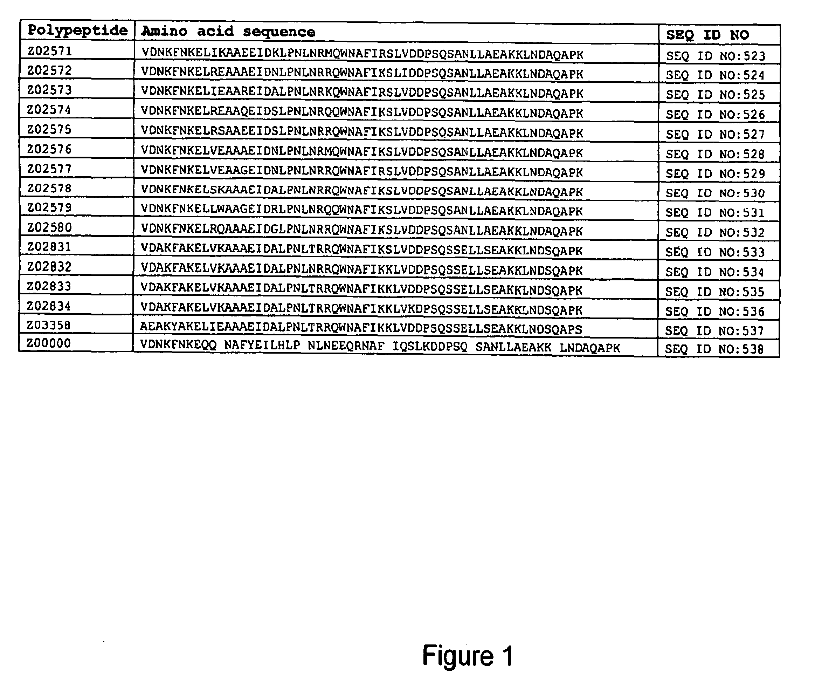

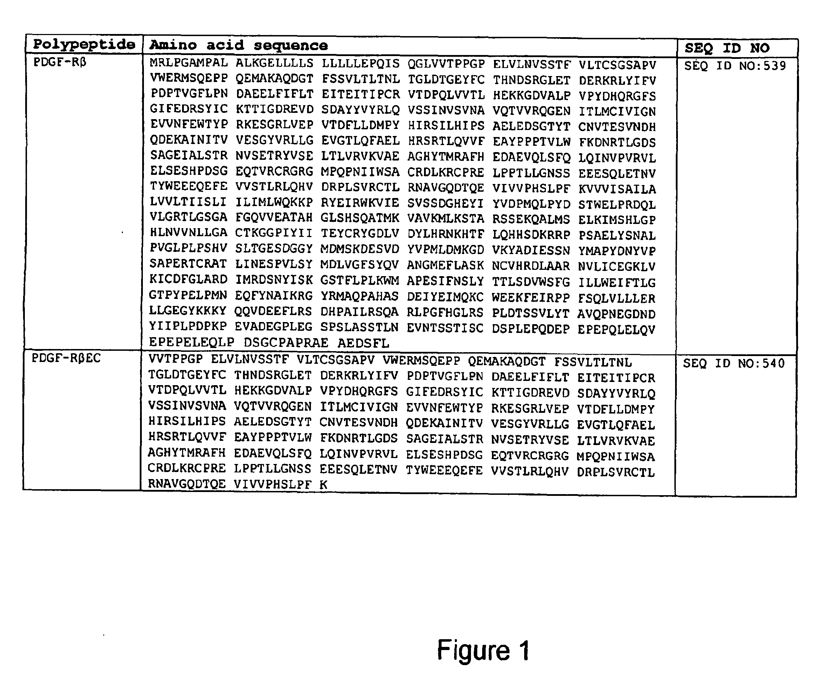

As described in detail in the experimental section to follow, the selection of PDGF-Rβ-binding variants has led to the identification of individual PDGF-Rβ-binding motif *(PBM*) sequences. These sequences constitute individual embodiments of the *PBM* sequence i) in the definition of PDGF-Rβ-binding polypeptides according to this aspect of the present invention. The sequences of individual PDGF-Rβ-binding motifs are presented in Figure 1 and as SEQ ID NO:1-179. In some embodiments of this aspect of the invention, the *PBM* sequence i) is selected from any one of SEQ ID NO:2-3, SEQ ID NO:5, SEQ ID NO:7, SEQ ID NO:11-12, SEQ ID NO:18-19, SEQ ID NO:38, SEQ ID NO:42, SEQ ID NO:44, SEQ ID NO:47, SEQ ID NO:60-62, SEQ ID NO:64, SEQ ID NO:67-68, SEQ ID NO:71-72, SEQ ID NO:78, SEQ ID NO:80-81, SEQ ID NO:83, SEQ ID NO:86, SEQ ID NO:91-92, SEQ ID NO:94-97, SEQ ID NO: 101-103, SEQ ID NO:105, SEQ ID NO:109, SEQ ID NO:111, SEQ ID NO:116, SEQ ID NO:119, SEQ ID NO:133, SEQ ID NO:137, SEQ ID NO:139-140, SEQ ID NO:149, SEQ ID NO:153, SEQ ID NO:160, SEQ ID NO:164, SEQ ID NO:170, SEQ ID NO:174 and SEQ ID NO:179.

In one embodiment, the PDGF-Rβ-binding polypeptide according to the invention comprises a platelet derived growth factor receptor beta binding motif, *PBM*, which motif consists of the amino acid sequence selected from

EX₂X₃X₄AAX₇EID X₁₁LPNLX₁₆RX₁₈QW NAFIX₂₅X₂₆LX₂₈D,

wherein, independently of each other,
X₂ is selected from L and R;
X₃ is selected from R, I, L, V, Q and S;
X₄ is selected from A, R, D, E, K and V;
X₇ is selected from A, Q and K;
X₁₁ is selected from A, R, E and S;
X₁₆ is selected from N and T;
X₁₈ is selected from R, G, K, S, T and V;
X₂₅ is selected from K, R, S and A;
X₂₆ is selected from S and K;
X₂₈ is selected from V, R, I and A.

The amino acid sequence of the *PBM* may for example be selected from any one of SEQ ID NO:2-3, SEQ ID NO:5, SEQ ID NO:7, SEQ ID NO:11, SEQ ID NO:60, SEQ ID NO:72, SEQ ID NO:78, SEQ ID NO:111 and SEQ ID NO:153.

In particular embodiments of the present invention, the *PBM* forms part of a three-helix bundle protein domain. For example, the *PBM* may essentially constitute two alpha helices with an interconnecting loop, within said three-helix bundle protein domain. In these embodiments, the PDGF-Rβ-binding polypeptide according to the invention may in particular comprise an amino acid sequence selected from:
i) K-[*PBM*]-DPSQSXₐX_{b}LLX_{c}EAKKLNDX_{d}Q;
   wherein
   [*PBM*] is a PDGF-Rβ-binding motif as defined above;
   Xₐ is selected from A and S;
   X_{b} is selected from N and E;
   X_{c} is selected from A and S;
   X_{d} is selected from A and S;
   and
ii) an amino acid sequence which has at least 80 % identity to any one of the sequences defined above.

Said amino acid sequence may have at least 82 %, such as at least 84 %, such as at least 86 %, such as at least 88 %, such as at least 90 %, such as at least 92 %, such as at least 94 %, such as at least 96 %, such as at least 98 % identity to the sequences defined above. The amino acid sequence may for example be selected from SEQ ID NO:180-358. In some embodiments of this aspect of the invention, the amino acid sequence i) may be selected from any one of SEQ ID NO:181-182, SEQ ID NO:184, SEQ ID NO:186, SEQ ID NO: 190-191, SEQ ID NO:197-198, SEQ ID NO:217, SEQ ID NO:221, SEQ ID NO:223, SEQ ID NO:226, SEQ ID NO:239-241, SEQ ID NO:243, SEQ ID NO:246-247, SEQ ID NO:250-251, SEQ ID NO:257, SEQ ID NO:259-260, SEQ ID NO:262, SEQ ID NO:265, SEQ ID NO:270-271, SEQ ID NO:273-276, SEQ ID NO:280-282, SEQ ID NO:284, SEQ ID NO:288, SEQ ID NO:290, SEQ ID NO:295, SEQ ID NO:298, SEQ ID NO:312, SEQ ID NO:316, SEQ ID NO:318-319, SEQ ID NO:328, SEQ ID NO:332, SEQ ID NO:339, SEQ ID NO:343, SEQ ID NO:343, SEQ ID NO:349, SEQ ID NO:353 and SEQ ID NO:358, and may in particular be selected from any one of SEQ ID NO:181-182, SEQ ID NO:184, SEQ ID NO:186, SEQ ID NO: 190, SEQ ID NO:239, SEQ ID NO:251, SEQ ID NO:257, SEQ ID NO:290, SEQ ID NO:332 and SEQ ID NO:358.

In particular embodiments of the invention, said three-helix bundle protein domain is selected from domains of bacterial receptor proteins. Nonlimiting examples of such domains are the five different three-helical domains of Protein A from *Staphylococcus aureus,* and derivatives thereof.

Thus, a PDGF-Rβ-binding polypeptide according the invention may comprise an amino acid sequence selected from:
ADNNFNK-[*PBM*]-DPSQSANLLSEAKKLNESQAPK (*PBM* within domain A of staphylococcal protein A);
ADNKFNK-[*PBM*]-DPSQSANLLAEAKKLNDAQAPK (*PBM* within domain B of staphylococcal protein A);
ADNKFNK-[*PBM*]-DPSVSKEILAEAKKLNDAQAPK (*PBM* within domain C of staphylococcal protein A);
ADAQQNNFNK-[*PBM*]-DPSQSTNVLGEAKKLNESQAPK (*PBM* within domain D of staphylococcal protein A);
AQHDE-[*PBM*]-DPSQSANVLGEAQKLNDSQAPK (*PBM* within domain E of staphylococcal protein A);
VDNKFNK-[*PBM*]-DPSQSANLLAEAKKLNDAQAPK (*PBM* within the protein Z derivative of domain B of staphylococcal protein A);
VDAKFAK-[*PBM*]-DPSQSSELLSEAKKLNDSQAPK (*PBM* within a variant of a protein Z derivative of domain B of staphylococcal protein A);
AEAKYAK-[*PBM*]-DPSQSSELLSEAKKLNDSQAPS (*PBM* within a variant of a protein Z derivative of domain B of staphylococcal protein A); and an amino acid sequence which has at least 80 % identity to any one of the sequences defined above, and wherein [*PBM*] is an PDGF-Rβ-binding motif as defined above. The inventive polypeptide may for example have a sequence which is at least 81 %, at least 83 %, at least 84 %, at least 86 %, at least 88 %, at least 90 %, at least 91 % at least 93 %, at least 95 %, at least 97 % or at least 98 % identical to the sequence described herein. In some embodiments of this aspect of the invention, said amino acid sequence comprises a sequence selected from any one of SEQ ID NO:359-537. The amino acid sequence may for example comprise a sequence selected from SEQ ID NO:360-361, SEQ ID NO:363, SEQ ID NO:365, SEQ ID NO:369-370, SEQ ID NO:376-377, SEQ ID NO:396, SEQ ID NO:400, SEQ ID NO:402, SEQ ID NO:405, SEQ ID NO:418-420, SEQ ID NO:422, SEQ ID NO:425-426, SEQ ID NO:429-430, SEQ ID NO:436, SEQ ID NO:438-439, SEQ ID NO:441, SEQ ID NO:444, SEQ ID NO:449-450, SEQ ID NO:452-455, SEQ ID NO:459-461, SEQ ID NO:463, SEQ ID NO:467, SEQ ID NO:469, SEQ ID NO:474, SEQ ID NO:477, SEQ ID NO:491, SEQ ID NO:495, SEQ ID NO:497-498, SEQ ID NO:507, SEQ ID NO:511, SEQ ID NO:518, SEQ ID NO:522, SEQ ID NO:528, SEQ ID NO:532 and SEQ ID NO:537, and may in particular comprise an amino acid sequence selected from SEQ ID NO:360-361, SEQ ID NO:363, SEQ ID NO:365, SEQ ID NO:369, SEQ ID NO:418, SEQ ID NO:430, SEQ ID NO:436, SEQ ID NO:469, SEQ ID NO:511 and SEQ ID NO:537.

A PDGF-Rβ-binding polypeptide according to any aspect of the invention may bind to PDGF-Rβ such that the K_{D} value of the interaction is at most 1 x 10⁻⁷ M, for example at most 1 x 10⁻⁸ M.

The polypeptides according to the invention are advantageous in that they bind well to PDGF-Rβ. The polypeptides may in particular bind to the extra-cellular domain of PDGF-Rβ. Typically, the polypeptides can be relatively short. By virtue of their small size, they are expected to exhibit a more efficient penetration in tumor tissue than antibodies, as well as to have better systemic circulation properties than monoclonal antibodies (which often have too long circulation times). Thus, they are considered suitable candidates for the development of molecular imaging agents. Additional possible applications include use in drug development and in screening procedures where specific imaging agents are desired in order to measure the outcome of treatment in *in vivo* models, and subsequently during clinical development. Molecular imaging provides a direct read-out efficacy of a pharmaceutical aimed at down-regulating a growth factor receptor, as well as for assessing the anti-tumor effect.

The skilled person will appreciate that various modifications and/or additions can be made to a polypeptide according to the invention in order to tailor the polypeptide to a specific application without departing from the scope of the present invention. For example, a PDGF-Rβ-binding polypeptide according to any aspect of the invention may be extended by C terminal and/or N terminal amino acids. Said extended polypeptide is a polypeptide which has additional amino acids residues at the very first and/or the very last position in the polypeptide chain, i.e. at the N- and/or C-terminus. The polypeptide may be extended by any suitable number of additional amino acid residues, for example at least one amino acid residue. Each additional amino acid residue may individually or collectively be added in order to, for example, improve production, purification, stabilization *in vivo* or *in vitro,* coupling, or detection of the polypeptide. Such additional amino acid residues may comprise one or more amino acid residues added for the purpose of chemical coupling. One example of this is the addition of a cysteine residue. Such additional amino acid residues may also provide a "tag" for purification or detection of the polypeptide such as a His₆ tag or a "myc" (c-myc) tag or a "FLAG" tag for interaction with antibodies specific to the tag.

The amino acid extensions discussed above may also provide one or more polypeptide domains with any desired function, such as the same binding function as the first PDGF-Rβ-binding domain, or another binding function, or an enzymatic function, toxic function (e.g. an immunotoxin), or a fluorescent signaling function, or combinations thereof. In such an extended polypeptide according to the invention, a PDGF-Rβ-binding polypeptide as described above is present as an PDGF-Rβ-binding domain to which additional peptides or proteins or other functional groups are coupled N- and/or C-terminally, or to any other residues (specifically or non-specifically). One example is an amino acid extension comprising the albumin-binding domain (ABD) of streptococcal protein G, or a derivative thereof. Such an ABD-extended polypeptide binds to serum albumin *in vivo,* and benefits from its longer half life, which increases the net half life of the polypeptide itself (see e.g. WO91/01743). Alternatively, the amino acid extension may comprise any other polypeptide with affinity for a serum protein.

Also covered by the present invention are modifications and/or additions to the polypeptide of the invention such as labels and/or therapeutic agents that are chemically conjugated or otherwise bound to the polypeptide.

The amino acid extensions, modifications and additions as discussed above may be coupled to the polypeptide of the invention by means of chemical conjugation (using known organic chemistry methods) or by any other means, such as expression of the polypeptide according to the invention as a fusion protein.

In one specific embodiment of the present invention, the polypeptide binds to the same epitope as PDGF-BB, or sufficiently close to it to block the binding of the ligand PDGF-BB to PDGF-Rβ. The polypeptide may for example be used to inhibit cell signaling by binding to a PDGF-Rβ on the cell surface. Such blocking of receptor function may be utilized to obtain a therapeutic effect. Alternatively, binding of the polypeptide to PDGF-Rβ may stimulate receptor activation by providing receptor dimerization.

In some embodiments of the invention, the polypeptide is present in multimeric form, comprising at least two PDGF-Rβ-binding polypeptide monomer units, the amino acid sequences of which may be the same or different. Multimeric forms of the polypeptide may be advantageous in that they may have enhanced binding properties. Preferred multimeric forms include dimeric forms. Such a dimeric form of the inventive polypeptide may for example be used to stimulate receptor activation. Multimeric forms of the polypeptides may comprise a suitable number of domains, each having a PDGF-Rβ-binding motif, and each forming a "monomer" within the multimer. These domains may all have the same amino acid sequence, but alternatively, they may have different amino acid sequences. The monomer units of a multimeric polypeptide may be joined by covalent coupling using known organic chemistry methods, or expressed as one or more fusion polypeptides, for example in a system for recombinant expression of polypeptides, or joined in any other fashion, either directly or via a linker, for example an amino acid linker.

In related aspects of the invention, there is provided a polynucleotide encoding a polypeptide as described above, as well as a method of producing such a polypeptide, the method comprising expression of the polynucleotide.

A polypeptide of the invention may be used as an alternative to conventional antibodies or low molecular weight substances in various medical, veterinary and diagnostic applications. The skilled addressee will understand that the polypeptides of the invention may be useful in any method which relies on affinity for PDGF-Rβ of a reagent. Thus, the inventive polypeptide may be used as a detection reagent, a capture reagent or a separation reagent in such methods, but also as a therapeutic or diagnostic agent in its own right or as a means for targeting other therapeutic or diagnostic agents, with direct (e.g. toxic molecules, toxins) or indirect (e.g. cancer vaccines, immunostimulatory molecules) effects on the PDGF-Rβ protein. Diagnostic applications include for example molecular imaging in order to reveal, diagnose and examine the presence of a disease, such as a tumor, *in vivo* in the body of a mammalian subject.

In one aspect of the invention, there is thus provided a PDGF-Rβ-binding polypeptide according to the invention for use as a medicament, for example for the treatment of a PDGF-Rβ-related condition. In this case, the PDGF-Rβ-binding polypeptide of the invention is used *in vivo* to obtain a therapeutic effect, for example by inhibiting cell signaling by binding to a PDGF-Rβ on a cell surface. There is also provided a PDGF-Rβ-binding polypeptide for use in diagnosis, such as in the diagnosis of a PDGF-Rβ-related condition.

In other aspects of the invention, the PDGF-Rβ-binding polypeptides may be used in targeting therapeutic or diagnostic agents, both *in vivo* and *in vitro*, to cells expressing PDGF-Rβ, particularly to cells which over-express PDGF-Rβ. There is thus provided a combination of a PDGF-Rβ-binding polypeptide according to the invention and a therapeutic agent. In one embodiment, said combination is used as a medicament, for example for the treatment of a PDGF-Rβ-related condition. There is moreover provided, in a related aspect of the invention, a combination of a PDGF-Rβ-binding polypeptide according to the invention and a diagnostic agent. Such a combination may be used in the diagnosis of a PDGF-Rβ-related condition, for example in molecular imaging of cells over-expressing PDGF-Rβ. In addition to the development of molecular imaging agents for the clinic, an application exists for specific preclinical imaging agents to measure the outcome of treatment in *in vivo* models and subsequently during clinical development. Molecular imaging should provide a direct read-out of the efficacy of a pharmaceutical aimed to down-regulate a receptor e.g. PDGF-Rβ, as well as for assessing the anti-tumor effect.

In a related aspect of the present invention, there is provided a Method of treatment of a PDGF-Rβ-related condition, comprising administering of a PDGF-Rβ-binding polypeptide or combination as described above to a mammalian subject in need thereof. Thus, in the inventive method of treatment, the subject is treated with a PDGF-Rβ-binding polypeptide or a combination according to the invention. In a more specific embodiment of said method, said binding of said PDGF-Rβ-binding polypeptide or said combination to a PDGF-Rβ of the subject inhibits or stimulates activation of the receptor. Said binding may for example inhibit receptor signaling. Also provided is a method for diagnosis of a PDGF-Rβ-related condition in a mammalian subject, comprising administering a PDGF-Rβ-binding polypeptide according to the invention, or a diagnostic combination as described above, to the subject for the purposes of obtaining a diagnosis.

In embodiments of the above described uses and methods of treatment, said condition may be selected from cancer disease, such as gliomas, sarcomas, and leukemias; vascular disorders, such as atherosclerosis, restenosis, pulmonary hypertension, and retinal diseases; fibrotic diseases, such as pulmonary fibrosis, liver cirrhosis, scleroderma, glomerulosclerosis, and cardiac fibrosis.

The terms "PDGF-Rβ-binding" and "binding affinity for PDGF-Rβ" as used in this specification refers to a property of a polypeptide which may be tested for example by the use of surface plasmon resonance technology, such as in a Biacore instrument (GE Healthcare). For example as described in the examples below, PDGF-Rβ-binding affinity may be tested in an experiment in which PDGF-Rβ, or a fragment of PDGF-Rβ such as the extracellular domain, is immobilized on a sensor chip of the instrument, and the sample containing the polypeptide to be tested is passed over the chip. Alternatively, the polypeptide to be tested is immobilized on a sensor chip of the instrument, and a sample containing PDGF-Rβ, or fragment thereof, is passed over the chip. The skilled person may then interpret the results obtained by such experiments to establish at least a qualitative measure of the binding of the polypeptide to PDGF-Rβ. If a quantitative measure is desired, for example to determine a K_{D} value for the interaction, surface plasmon resonance methods may also be used. Binding values may for example be defined in a Biacore 2000 instrument (GE Healthcare). PDGF-Rβ is immobilized on a sensor chip of the measurement, and samples of the polypeptide whose affinity is to be determined are prepared by serial dilution and injected over the chip. K_{D} values may then be calculated from the results using for example the 1:1 Langmuir binding model of the BIAevaluation 4.1 software provided by the instrument manufacturer. The PDGF-Rβ or fragment thereof may for example comprise the amino acid sequence represented by SEQ ID NO:540 (PDGF-Rβ extra-cellular domain) or SEQ ID NO: 539 (PDGF-Rβ). The extracellular domain of recombinant human PDGF-Rβ (amino acid residue 1-530, Gronwald *et al*, 1998, PNAS 85) provided by R&D Systems, #385-PR/CF may for example be used.

### Brief description of the figures

Figure 1 is a listing of the amino acid sequences of examples of PDGF-Rβ-binding motifs comprised in PDGF-Rβ-binding polypeptides of the invention (SEQ ID NO:1-179), examples of 49 amino acid residues long PDGF-Rβ-binding polypeptides according to the invention (SEQ ID NO:180-358) and examples of 58 amino acid residues long PDGF-Rβ-binding polypeptides according to the invention (SEQ ID NO:359-537), as well as the sequences of protein Z (SEQ ID NO:538), the Swiss-Prot entry P09619 of human PDGF-Rβ (amino acid residues 1-1106, SEQ ID NO:539) and the Swiss-Prot entry P09619 of the extra-cellular domain of human PDGF-Rβ (amino acid residues 33-531 of PDGF-Rβ, SEQ ID NO:540).

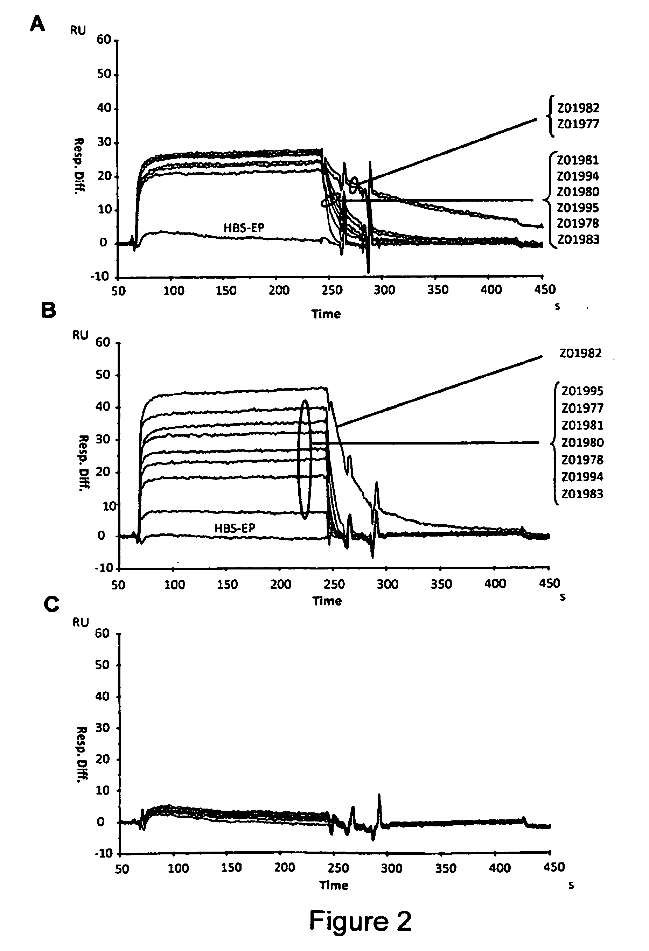

Figures 2A-C show the result of binding analysis performed in a Biacore instrument for investigating the binding of different monomeric Z variants to PDGF receptors. Eight different Z variants were injected in duplicates over Biacore chip surfaces with immobilized PDGF receptors. A) shows injection over human PDGF-Rβ, B) shows injection over murine PDGF-Rβ, and C) shows injection over human PDGF-Rα. The Z variants are listed in two groups, the individual Z variants in each Z variant group corresponding top-down to individual curves in each group of curves.

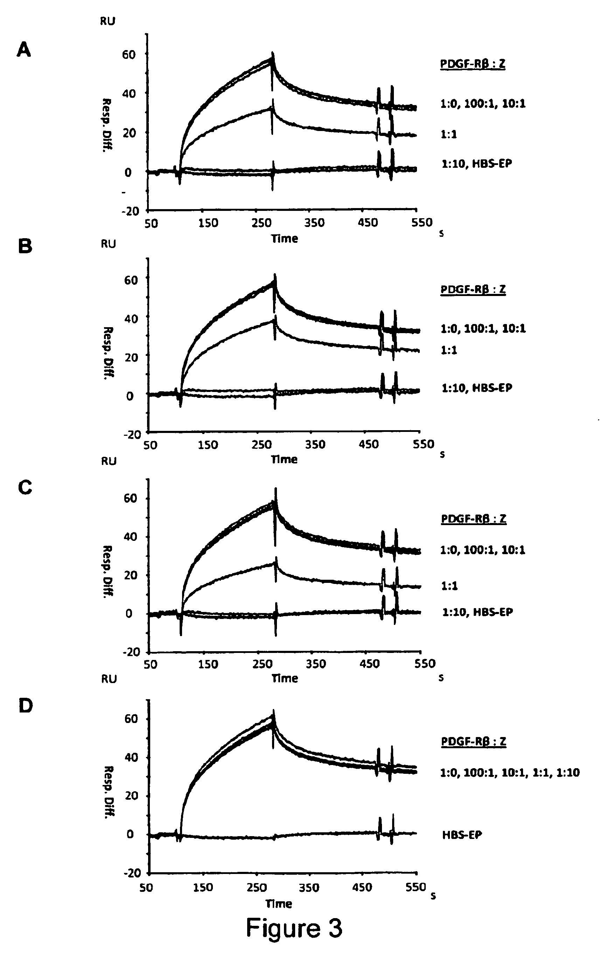

Figures 3A-D show the result of an epitope test of Z variant molecules on PDGF-Rβ. Analytes containing varying concentrations of Z variant molecules and a constant concentration of PDGF-Rβ were injected over immobilised PDGF-BB. A) shows Z01977, B) shows Z01980, C) shows Z01982 and D) shows a negative control (ZAβ). The relative concentrations of PDGF-Rβ and Z variant are shown in relation to each group of curves. Analytes containing higher Z variant molecule concentrations and buffer HBS-EP are seen near the baseline.

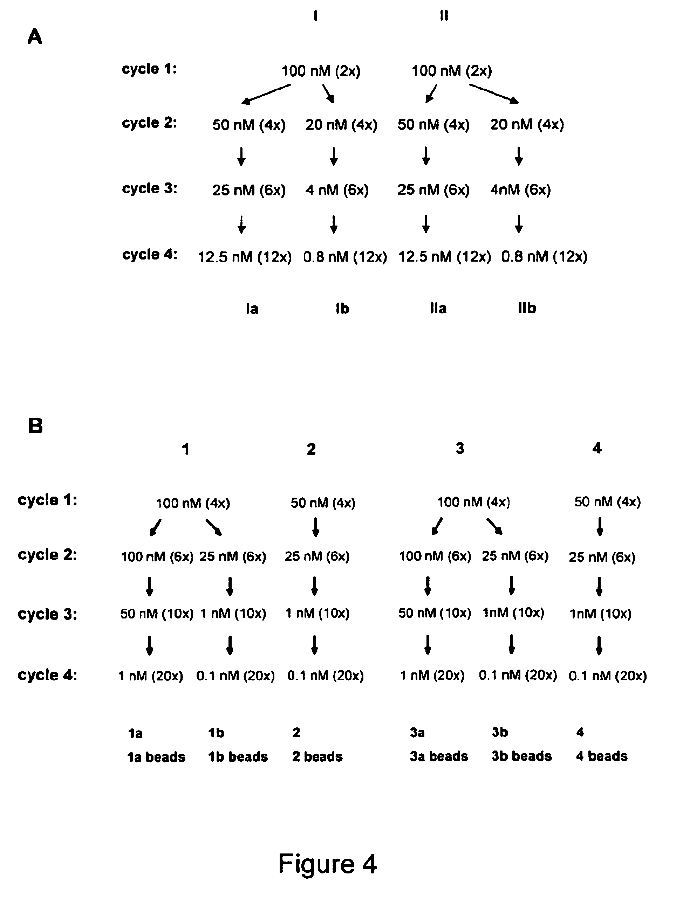

Figure 4 is an overview of the selections performed in Example 1 (A) and in Example 4 (B) showing target concentrations (nM) and number of washes (in parenthesis). Selection from the first library, (A), was performed in four cycles initially divided into two tracks (I and II). In the second selection cycle, these were further divided into two tracks (Ia and Ib, IIa and IIb), resulting in totally four tracks. Track Ia and Ib selections were performed against non-biotinylated PDGF-Rβ and track IIa and IIb selections were performed against biotinylated PDGF-Rβ. Selection from the maturated library, (B), was initially performed in four tracks. These were further divided in cycle two, resulting in totally six tracks. Selection was performed in four cycles.

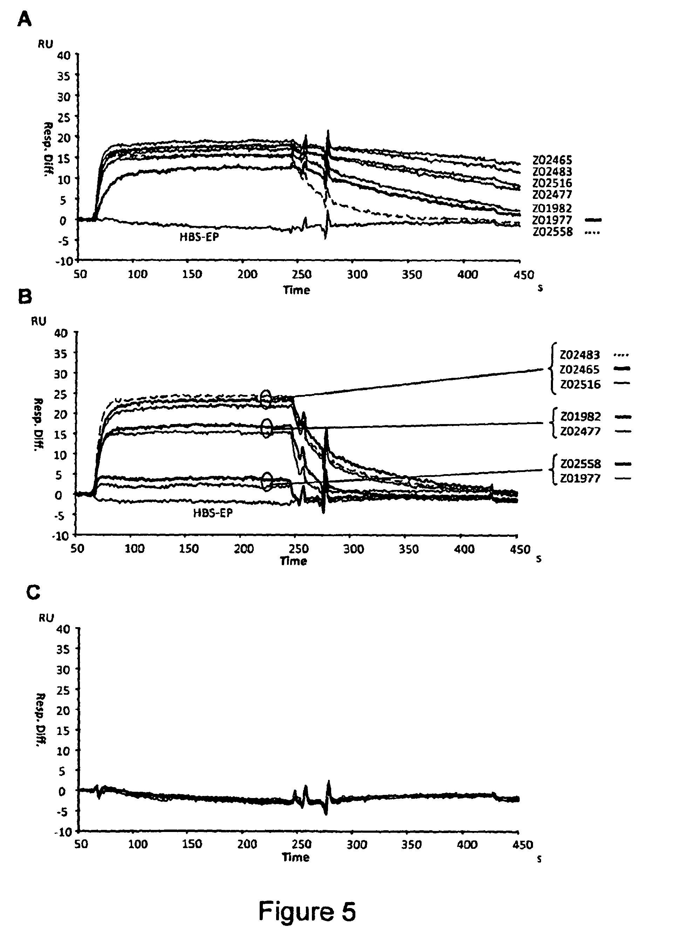

Figures 5A-C show the result of a binding analysis performed in a Biacore instrument. Different monomeric Z variants from the maturated library (Example 4) were tested for their binding to PDGF receptors. A) shows binding to human PDGF-Rβ, B) shows binding to murine PDGF-Rβ, and C) shows binding to human PDGF-Rα. Five different Z variants were injected in duplicates or triplicates over Biacore chip surfaces with immobilized PDGF receptors; Z02558, Z02516, Z02483, Z02477 and Z02465. Two Z variants from the selection in Example 1 were run as references (Z01977 and Z01982), as well as an injection of running buffer HBS-EP.

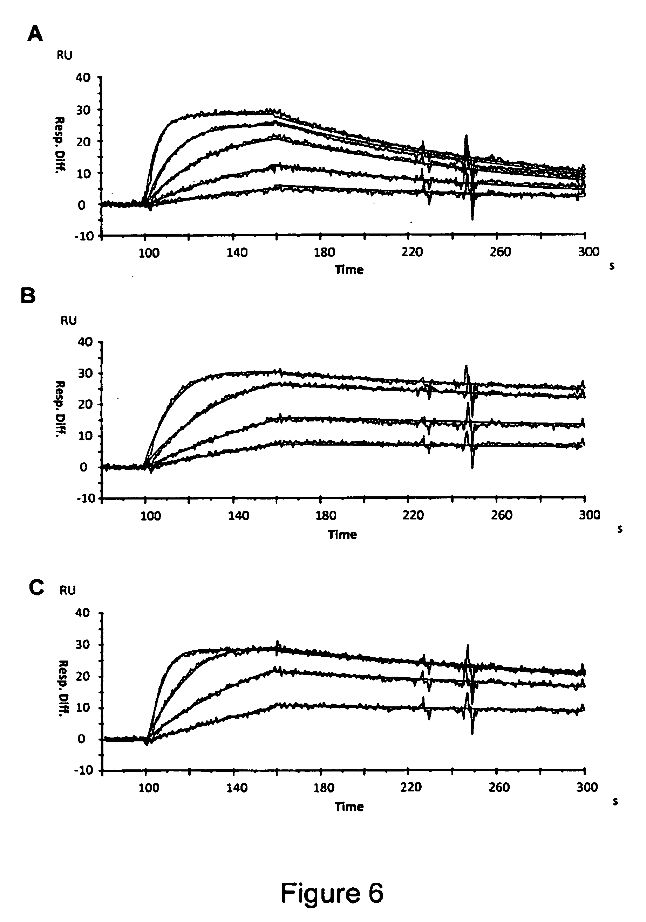

Figures 6A-C show sensorgrams from kinetic experiments performed in a Biacore instrument with curve fitting in a Langmuir 1:1 binding model. Varying concentrations of the different Z variants A) Z01982, B) Z02465 and C) Z02483 were injected over immobilized human PDGF-Rβ. The straight curves represent the fitted model.

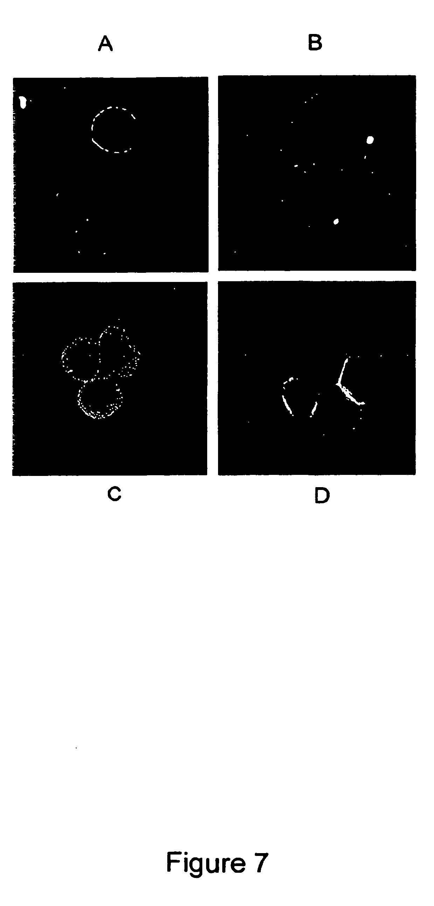

Figures 7A-D show images from the immunofluorescence experiment described in Example 5. The images show PDGF-Rβ-expressing AU565 cells stained with different antibodies or Z variants: A) goat anti-PDGF-Rβ antibody, B) or His₆-(Z02465)₂-Cys, C) His₆-(Z02483)₂-Cys, and D) His₆-(Z02516)₂-Cys.

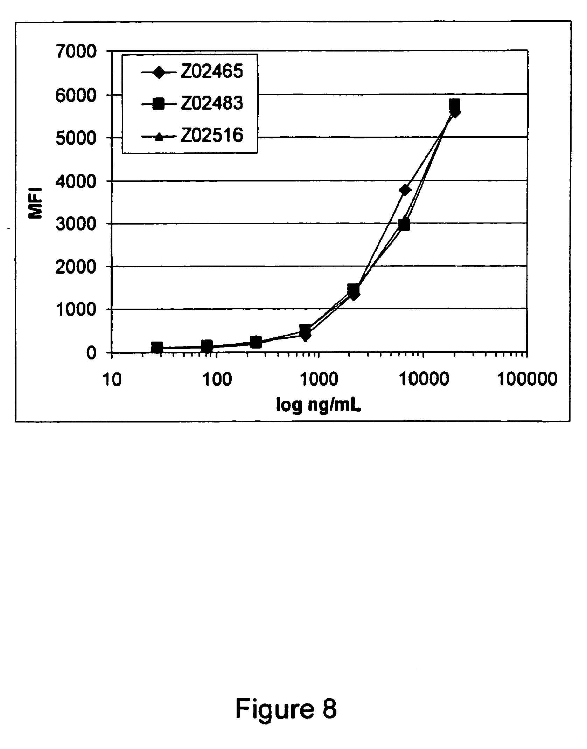

Figure 8 shows the result from the flow cytometry experiment described in Example 5. The Figure shows titration of Alexa Fluor® 647 conjugated His₆-(Z02465)₂-Cys (◆), His6-(Z02483)₂-Cys (■) and His6-(Z02516)₂-Cys (▲) on PDGF-Rβ-expressing AU565 cells. Mean fluorescence intensity was plotted against log concentration (ng/ml) of Z variants.

### Examples

The following materials where used throughout this work except where otherwise noted.
- *Escherichia coli* strain RR1ΔM15 (Rüther, Nucleic Acids Res 10:5765-5772, 1982)
- The extracellular domain of recombinant human PDGF-Rβ with a C-terminal Fc fusion and His₆-tag (R&D Systems, #385-PR/CF)
- Murine PDGF-Rβ-Fc (R&D Systems, #1042-PR/CF)
- Human PDGF-sRα (R&D Systems, #322-PR/CF)
- PDGF-BB (R&D Systems #220-BB/CF)

### Example 1: Selection and screening of PDGF-Rβ-binding polypeptides

### Materials and methods

Biotinylation of target protein: The extracellular domain of recombinant human PDGF-Rβ was biotinylated with a 10x molar excess of EZ-link Sulfo-NHS-LC-Biotin (Pierce #21327) in PBS (10 mM phosphate, 137 mM NaCl, 2.68 mM KCl, pH 7.4). In order to remove any excess biotin, a buffer exchange was made on desalting PD-10 columns (GE Healthcare #17-0851-01), pre-equilibrated with PBS according to the manufacturer's instructions.

### Preparation of Z00000-coupled and Fc-coated streptavidin beads:

Biotinylated Z00000 (Affibody AB, #10.0623.02.0005), i.e. protein Z (SEQ ID NO:538) as described in Nilsson et al, Prot Eng 1:107-113, 1987, was coupled to streptavidin coated magnetic beads (Dynabeads® M-280 Streptavidin, Dynal #112.06). 15 µg (Z00000)₂-Cys-biotin was added per mg beads, and the beads were incubated for 30 minutes at room temperature (RT). The beads were washed with PBS-T 0.1 (PBS supplemented with 0.1 % Tween 20). The Z00000 coupled beads were thereafter coated with polyclonal human IgG1-Fc (Jackson Immuno Research # 009-000-008) by end-over-end incubation for 1 hour at RT using 4 µg Fc per mg Z00000 coupled beads. The beads were washed with PBS-T 0.01 prior to use in the pre-selection described below.

Phage display selection of PDGF-Rβ-binding polypeptides: A library of random variants of protein Z displayed on bacteriophage, constructed in phagemid pAffi1/pAY00065 as described in Grönwall et al, J Biotechnol 128:162-183 (2007), was used to select PDGF-Rβ-binding polypeptides. Selection was performed in four cycles initially divided into two tracks (I and II). In the second selection cycle, these were further divided into two tracks, resulting in totally four tracks (Ia=high target concentration, Ib=low target concentration, IIa=high target concentration, and IIb=low target concentration). Tracks Ia and Ib were performed against non-biotinylated PDGF-Rβ, and tracks IIa and IIb against biotinylated PDGF-Rβ. An overview of the selection strategy is shown in Figure 4A.

Phage library stock was prepared according to previously described procedures (Nord et al, Nat Biotech 15:772-777 (1997); Hansson et al, Immunotechnology 4:237-252 (1999)). Prior to selection, the phage stock was twice precipitated in PEG/NaCl (20 % polyethyleneglycol (PEG), 2.5 M NaCl) and dissolved in 1 ml selection buffer (0.1 % gelatin in PBS-T 0.1). In order to reduce the amount of background binders, a pre-selection was performed by incubation of phage stock with biotinylated Z00000-coupled streptavidin beads coated with Fc protein (track I) or with streptavidin beads (track II) for 1 hour at RT. All tubes and beads used in the selection were pre-blocked with selection buffer.

In cycle 1 of the selection, the supernatant resulting from the pre-selection was mixed with 100 nM PDGF-Rβ (track I) or 100 nM biotinylated PDGF-Rβ (track II), followed by incubation under continuous rotation for 2 hours at RT. Target-phage complexes were captured on streptavidin beads via biotinylated Z00000 (0.5 mg beads, 20 minutes, track I) or directly to streptavidin beads (0.77 mg beads, 15 minutes, track II). The bead-bound phages were washed 2 times with 1000 µl PBS-T 0.1. After the wash, the bound phages were eluted with 500 µl 0.1 M glycine-HCl, pH 2.2 followed by immediate neutralization with 50 µl Tris-HCl, pH 8.0 and 450 µl PBS. Selected phage particles were amplified as described below and new phage stocks were prepared between each cycle. Phage stock, i.e. phages entering the selection cycle, and eluted phage particles were titrated after each selection cycle.

In cycle 2, selection tracks I and II were respectively split into selection tracks Ia and Ib, and tracks IIa and IIb. Thus, the newly prepared phage stocks were incubated with 50 nM (Ia) or 20 nM (Ib) PDGF-Rβ in selection buffer under continuous rotation for 2 hours at RT. Phage particle-target complexes were captured as in cycle 1 with 0.25 mg (Ia) or 0.1 mg (Ib) beads for 20 (Ia) or 15 (Ib) minutes. Similarly, phage stocks were incubated with 50 nM (IIa) or 20 nM (IIb) biotinylated PDGF-Rβ in selection buffer, followed by capturing with 0.4 mg (IIa) or 0.15 mg (IIb) beads. The wash was performed as in cycle 1 but with 4 washes. Elution was performed as in cycle 1.

In cycle 3, phage stocks were incubated with 25 nM (Ia) or 4 nM (Ib) PDGF-Rβ under continuous rotation as described above, followed by capturing with 0.15 mg (Ia) or 0.1 mg (Ib) beads. Similarly, phage stocks were incubated with 25 nM (IIa) or 4 nM (IIb) biotinylated PDGF-Rβ and captured with 0,2 mg (IIa) or 0,1 mg (IIb) beads. The wash was performed as in cycle 2 but with 6 washes. Elution was performed as in cycle 1.

In the last selection cycle, phage stocks were incubated with 12.5 nM (Ia) or 0.8 nM (Ib) PDGF-Rβ under continuous rotation and captured with 0.15 mg (Ia) or 0.1 mg (Ib) beads. Similarly, phage stocks were incubated with 12.5 nM (IIa) or 0.8 nM (IIb) biotinylated PDGF-Rβ under continuous rotation and captured with 0.2 mg (IIa) or 0.1 mg (IIb) beads. The wash was performed as in cycle 3 but with 12 washes. Elution was performed as in cycle 1.

Amplification of phage particles: Log phase *E*. *coli* RR1ΔM15 cells were infected with 950 µl eluted phage particles for 20 min at 37 °C after each cycle of selection. The phages that were still bead-bound after elution were similarly amplified. After 20 minutes of incubation at 37 °C, the cells infected with eluted phage and the cells infected with bead-bound phage originating from the same selection cycle and track, were pooled and pelleted by centrifugation. The pellet was dissolved in a small volume of TSB-YE medium (30 g/l TSB, 5 g/l yeast extract) and spread on tryptone yeast extract plates (TYE: 15 g/l agar, 10 g/l tryptone water (Merck), 5 g/l yeast extract, 3 g/l NaCl, 2 % glucose and 0.1 g/l ampicillin), followed by incubation overnight at 37 °C. In the final selection cycle, bacteria were diluted before spreading onto TYE plates in order to form single colonies to be used in ELISA screening.

Phage infected bacteria grown on the TYE plates were re-suspended in TSB medium. An amount of the re-suspended cells was prepared as glycerol stock and stored at -20 °C. Suspended cells corresponding to 100-1000 times the number of eluted phage were inoculated in TSB-YE medium supplemented with 2 % glucose and 100 µg/ml ampicillin. The cells were grown to log phase at 37°C. An amount of cells corresponding to the same amount of cells used for inoculation was infected with a 20x excess of M13K07 helper phage (New England Biolabs #NO315S) during 30 min at 37 °C. Cells were pelleted by centrifugation, re-suspended in TSB-YE medium supplemented with 100 µM IPTG (1 M isopropyl-β-D-1-thiogalactopyranoside), 25 µg/ml kanamycin and 100 µg/ml ampicillin, and grown overnight at 30 °C. The overnight cultures were centrifuged, and phage particles in the supernatant were precipitated twice with PEG/NaCl buffer. Finally, the phages were re-suspended in selection buffer before entering the next selection cycle.

ELISA screening of Z variants: To test if the Z variant molecules could indeed interact with the PDGF-Rβ, an ELISA was performed. The Z variants were produced by inoculating single colonies, prepared as described above, in 1 ml TSB-YE medium supplemented with 100 µg/ml ampicillin and 0.1 mM IPTG in deep-well plates (Nunc #278752). The plates were incubated for 18-24 h at 37°C. After incubation, replicate plates were made by transferring a small fraction of each culture to 96-well plates with 15 % glycerol for storage at -20 °C. Remaining cells were pelleted by centrifugation, re-suspended in 300 µl PBS-T 0.05 (PBS supplemented with 0.05 % Tween 20) and frozen at -80 °C to release the periplasmic fraction of the cells. Frozen samples were thawed in a water bath and cells were pelleted by centrifugation. The periplasmic supernatant contained the Z variants as fusions to an albumin binding domain (ABD, GA3 of protein G from *Streptococcus* strain G148), expressed as AQHDEALE-[Z#####]-VDYV-[ABD]-YVPG (Grönwall *et al, supra*). Z##### refers to individual Z variants.

Half area 96 well ELISA plates (Costar #3690) were coated with 50 µl/well of coating buffer (50 mM sodium carbonate, pH 9.6) containing 6 µg/ml human serum albumin (HSA, Sigma #A3782), and incubated over night. The HSA solution was poured off and the wells were blocked with 100 µl of PBS-T 0.1 supplemented with 2 % non-fat dry milk solution (Semper AB) for 1 h at RT. The blocking solution was discarded and 50 µl of periplasmic solution were added to the wells and incubated for 1.5 h at RT under slow shaking. The supernatants were poured off and the wells were washed 4 times with PBS-T 0.05. 50 µl of a mixture containing both biotinylated and non-biotinylated PDGF-Rβ at a concentration of 4.5 µg/ml in PBS-T 0.1 was added to each well. The plates were incubated for 1.5 h at RT followed by wash 4x in PBS-T 0.05. IgG-Fc control plates were prepared by addition of 3 µg/ml Fc from human IgG in 50 µl PBS-T to the wells. The control plates were incubated for 1.5 h at RT and washed as described above. An antibody against Fc (DAKO, #P0214), labeled with horseradish peroxidase and diluted 1:4000 in PBS-T 0.1, was added to the wells. After washing as described above, 50 µl ImmunoPure TMB substrate (Pierce #34021) was added to the wells and the plates were treated according to the manufacturer's recommendations. All steps from blocking to reading were performed in a Tecan Genesis Freedom 200 robot (Tecan Group LTD). Absorbance of the wells was read at 450 nm in an ELISA reader Tecan Ultra 384 (Tecan) and evaluated with Magellan v. 5.0 software (Tecan).

Sequencing: From the ELISA screening, clones regarded as positive were picked for sequencing. PCR fragments were amplified from single colonies using a standard PCR program and the primers AFFI-21 (5'-tgcttccggctcgtatgttgtgtg) and AFFI-22 (5'-cggaaccagagccaccaccgg). Sequencing of amplified fragments was performed using the biotinylated oligonucleotide AFFI-72 (5'-biotin-cggaaccagagccaccaccgg) and a BigDye® Terminator v3.1 Cycle Sequencing Kit (Applied Biosystems), used in accordance with the manufacturer's protocol. The sequencing reactions were purified by binding to magnetic streptavidin coated beads using a Magnatrix 8000 (Magnetic Biosolution), and analyzed on ABI PRISM® 3100 Genetic Analyzer (PE Applied Biosystems). The sequencing results were imported and analyzed with an ALD LIMS Nautilus™ 2003 R2 B3 software (Thermo Electronics Corp.).

Subcloning of Z variants: Monomeric and dimeric Z variants were amplified from pAffi1/pAY00065 vectors. A PCR was performed using different primer pairs and the resulting gene fragments were purified and hybridized in ligase buffer.

The hybridized gene fragments were subcloned in the pAY01448 vector, providing an N-terminal His₆ tag (His₆-Z#####), and in the pAY01449 vector, providing an N-terminal His₆ tag and a C-terminal cysteine (His₆-(Z#####)₂-Cys). The PDGF-Rβ-binding Z variants were subcloned as monomers in pAY01448 and as dimers in pAY01449, and the constructs encoded by the expression vectors were MGSSHHHHHHLQ-[Z#####]-VD for the monomers and MGSSHHHHHHLQ-[Z#####][Z#####]-VDC for the dimers. For the dimers, a three parts ligation was used for insertion of both insert fragments into the vector at the same step. Hybridized gene fragments and Accl-digested and dephosphorylated expression vectors were ligated in ligase buffer and electroporated into electrocompetent *E*. *coli* TOP1 0 cells. The transformed cells were spread on TBAB plates (30 g/l tryptose blood agar base) supplemented with 50 µg/ml of kanamycin, followed by incubation at 37 °C overnight. The colonies were screened using PCR and the lengths of the PCR fragments were verified on agarose gels. To verify the sequences, sequencing was performed with BigDye® Terminator v3.1 Cycle Sequencing Kit (Applied Biosystems), followed by analysis in an ABI PRISM® 3100 Genetic Analyzer (PE Applied Biosystems) and evaluation using Sequencher™ 4.0.5. software (Gene Codes Corporation).

Plasmid DNA stock was prepared from the sequenced clones and deposited in -80 °C. In addition, *E. coli* BL21 (DE3) cells were transformed with the plasmids, either through electroporation (monomeric constructs) or through transformation of chemically competent cells (dimeric constructs).

### Results

Phage display selection of PDGF-Rβ-binding polypeptides: Four cycles of phage display selections were run against non-biotinylated (track I) and biotinylated (track II) human PDGF-Rβ fused to the Fc part of IgG. Phage particle-target complexes were captured onto streptavidin-coated beads with (I) or without (II) bead-coupled Z00000-biotin. The four selection cycles were performed with Ia) non-biotinylated target, high target concentration, Ib) non-biotinylated target, low target concentration, IIa) biotinylated target, high target concentration, and IIb) biotinylated target, low target concentration. For each selection cycle, the number of washes was increased. The phage particle titers and yields were calculated after each selection cycle. The phage particle yield (phage particles out/phage particles in) increased for each cycle (except the second one), indicating an enrichment in target binding clones.

ELISA screening of Z variants: The clones obtained after four cycles of selection were produced in 96-well plates and screened for PDGF-Rβ-binding activity in an ELISA. In parallel, binding to Fc was tested for each clone. In total, 93 clones from each selection track (Ia, Ib, IIa, IIb) were screened. The absorbance measurements showed many clearly PDGF-Rβ positive clones and a few Fc positive clones. A Z variant molecule from selections against CD33 was used as negative and positive controls. The positive control was detected with human CD33-Fc and the negative control was not detected. Sequencing: Sequencing was performed for the clones with the highest absorbance values against PDGF-Rβ and with low absorbance values against Fc, i.e. values equal to values of background absorbance, in the ELISA screening. In total, 147 PDGF-Rβ positive clones were run. Most of the clones were found in several copies. However, eleven new Z variants were identified. Each variant was given a unique identification number #####, and individual variants are referred to as Z#####. The amino acid sequences of the 58 amino acid residues long Z variants are listed in Figure 1 and in the sequence listing as SEQ ID NO:359-369. The deduced PDGF-Rβ-binding motifs of these Z variants are listed in Figure 1 and in the sequence listing as SEQ ID NO:1-11. The amino acid sequences of the 49 amino acid residues long polypeptides predicted to constitute the complete three-helix bundle within each of these Z variants are listed in Figure 1 and in the sequence listing as SEQ ID NO: 180-190.

The unique sequences were grouped together in a cluster of similar sequences with an internal dissimilarity lower than approximately 25 % (results not shown). The sequence similarities suggest that the Z variants bind to the same binding surface on the PDGF-Rβ receptor.

Subcloning of Z variants: The unique clones were chosen for subcloning in the expression vectors pAY01448 and pAY01449 as monomers and dimers, respectively. The cloning resulted in eight monomers (Z01977, Z01978, Z01980, Z01981, Z01982, Z01983, Z01994 and Z01995) and seven dimers (dimers of Z01976, Z01977, Z01980, Z01982, Z01983, Z01994 and Z01995).

### Example 2: Production and characterization of Z variants

### Materials and methods

Protein expression and purification: Transformed *E*. *coli* BL21 (DE3) cultures as subcloned in Example 1 were grown to an optical density of approximately 1 (diluted 10x) and protein expression was induced by addition of 1 M IPTG (0.5 ml/l culture). Cultures were harvested 5 h after induction, by 20 min of centrifugation at 15900 g. The supernatants were discarded and the cell pellets were collected and stored at -20 °C prior to further use.

The PDGF-Rβ-binding Z variants were purified from cell pellets under denatured conditions on Ni-NTA Superflow Columns (Qiagen), and buffer was exchanged to PBS using PD-10 columns (GE Healthcare). Expression levels of soluble and unsoluble proteins were analyzed using SDS-PAGE by ocular determination of Coomassie stained gels. Purified Z variants that were not to be used directly were aliquoted and stored at -80 °C.

Protein characterization: The concentration of purified Z variants (in His₆-Z##### form and in His₆-(Z#####)₂-Cys form) was determined by absorbance measurements at 280 nm using theoretical extinction coefficients. The purity was estimated by SDS-PAGE analysis on 10 wells 4-12% NuPAGE™ gels (Invitrogen) using Coomassie blue staining. To verify the identity and to determine the molecular weights of purified Z variants, LC/MS-analyses were performed on an Agilent 1100 LC/MSD system (Agilent Technologies). CD analysis: The purified Z variants were thawed and diluted to 0.5 mg/ml in PBS. For each diluted Z variant, a CD spectrum at 250-195 nm was obtained at 37 °C. In addition, a variable temperature measurement (VTM) was performed to determine the melting temperature (Tm). In the VTM, the absorbance was measured at 221 nm while the temperature was raised from 20 to 90 °C, with a temperature slope of 5 °C/min. The CD measurements were performed on a Jasco J-810 spectropolarimeter (Jasco Scandinavia AB) using a cell with an optical path-length of 1 mm.

Biacore binding analysis: The interactions of eight His₆-tagged monomeric PDGF-Rβ-binding Z variants with human PDGF-Rβ, murine PDGF-Rβ-Fc and human PDGF-sRα were analyzed in a Biacore instrument (GE Healthcare). The three receptors were immobilized in different flow cells on the carboxylated dextran layer of a CM5 chip surface (GE Healthcare). The immobilization was performed using amine coupling chemistry according to the manufacturer's protocol. One flow cell surface on the chip was activated and deactivated for use as blank during analyte injections. The analytes, i.e. Z variants diluted in HBS-EP running buffer (GE Healthcare) to a final concentration of 5 µM, were injected in random order in duplicates at a flow-rate of 10 µl/minute for 3 minutes. After 3 minutes of dissociation, the surfaces were regenerated with one injection of 25 mM HCl. The results were analyzed in BiaEvaluation software (GE Healthcare). Curves of the blank surface were subtracted from the curves of the ligand surfaces.

Epitope test: The interaction of the human PDGF-Rβ receptor with its natural ligand PDGF-BB in the presence of three different Z variants (Z01977, Z01980 and Z01982 in His₆-Z##### form) was analyzed in a Biacore instrument (GE Healthcare). Human PDGF-BB was immobilized on the carboxylated dextran layer of two flow cells on a Biacore CM5 chip surface. The ligand density was different in the two flow cells. Analytes containing 100 nM PDGF-Rβ mixed with varying concentrations of monomeric His₆-tagged Z variants were prepared by dilution in the running buffer HBS-EP. A Z variant specifc for an irrelevant protein (amyloid β peptide, Aβ) was used as a negative control. The analytes were injected at a flow-rate of 10 µl/minute for 3 minutes. After 3 minutes of dissociation, the surfaces were regenerated with two injections of 10 mM HCl. The results were analyzed as described above. Dot blot analysis: Seven PDGF-Rβ-binding Z variants were tested for specificity by dot blot analysis. The Z variants were tested against alpha-2 macroglobulin (MP biomedicals/Cappel, #55833), alpha-1 acid glycoprotein (RDI, #RD)-SCP-153-1), alpha-1 antichymotrypsin (RDI, #RDI-SCP-159-0), alpha-1-antitrypsin (RDI, #RDI-SCP-165-5), C3 complement (RDI, #RDI-SCP-150-0), C4 complement (RDI, #RDI-SCP-151-0), fibrinogen (Enzyme research, #031015E), haptoglobulin (RDI, #RDI-SCP-119-1), hemopexin (Agilent), human IgG1 Fc, polyclonal (Jackson Immuno Research, #009-000-008), holo-transferrin (Sigma, #T0665), human IgA (Bethyl, #P80-102), human IgE (Fitzgerald, #30 A101), human IgG (Sigma, #G4386), human IgM (Sigma, #18260), human PDGF-sRα (R&D Systems, #322-PR/CF), human Serum Albumine (HSA, Sigma, #A3782), murine PDGF-Rβ-Fc (R&D Systems, #1042-PR/CF), neutravidin (Pierce, #31000), streptavidin (Pierce, #21122), transthyretin (Sigma, #P1742), and human PDGF-Rβ-Fc. Nitrocellulose membranes (Invitrogen) were dotted with 1 µl of each protein at a concentration of 0.1 mg/ml. The membranes were blocked for 1 h in PBS supplemented with 0.5 % casein (blocking solution) at RT. After removal of the solution, the membranes were incubated for 1 h with 2 µg/ml of different dimeric Z variants, with N terminal His₆ tags and C terminal cysteines. The membranes were washed 4 x 5 minutes in PBS-T 0.1. The Z variants were detected with a polyclonal goat IgG against an epitope common to all Z variants (Affibody AB, #20.1000.01.0005). This goat anti-Z IgG, diluted to 1 µg/ml in blocking solution, was added to the membranes which were incubated for 1 h at RT. After washing, an antibody against goat IgG conjugated to HRP (DAKO #P0449), diluted 1:10000 in blocking solution, was added to the membranes, followed by incubation of the membranes for 1 h at RT. The membranes were washed, rinsed in PBS and soaked with Supersignal (Pierce #34075). Light emissions were photographed with a Chemilmager 5500 (Alpha Innotech Corp.).

### Results

Protein production: Both monomeric (in His₆-Z##### form) and dimeric (in His₆-(Z#####)₂-CYS form) Z variant molecules yielded acceptable expression levels of soluble gene product. The purity of produced batches was assessed by SDS-PAGE analysis. The purity was estimated to exceed 95 % for the monomeric molecules and to be approximately 90 % for dimeric molecules.

The LC/MS analysis verified the correct molecular weight for all Z variant molecules.

CD analysis: In the CD analysis, the spectrum measurements were performed at 37 °C. At that temperature, the a-helical structures of the Z variants molecules had attained a partly unfolded state. This result was also verified in the variable temperature measurements where the melting temperatures (Tm) were determined (Table 1).

**Table 1. Melting temperatures for a number of Z variants**

| **Z variant** | **Tm (°C)** |
|---|---|
| His₆-Z01977 | 30 |
| His₆-Z01978 | 43 |
| His₆-ZO1980 | 35 |
| His₆-Z01981 | 37 |
| His₆-Z01982 | 37 |
| His₆-Z01983 | 37 |
| His₆-Z01994 | 30 |
| His₆-Z01995 | 36 |
| His₆-(Z01976)₂-Cys | 34 |
| His₆-(ZO1977)₂-Cys | 35 |
| His₆-(Z01980)₂-Cys | 35 |
| His₆-(Z01982)₂-Cys | 41 |
| His₆-(Z01983)₂-Cys | 38 |
| His₆-(Z01994)₂-Cys | 31 |
| His₆-(Z01995)₂-Cys | 37 |

Biacore binding analysis: The binding of eight monomeric Z variants (Z01977, Z01978, Z01980, Z01981, Z01982, Z01983, Z01994 and Z01995) to human and murine PDGF-Rβ, as well as to human PDGF-sRa, was tested in a Biacore instrument by injecting the Z variants over surfaces containing the three receptors. The ligand immobilization levels on the surfaces were: human PDGF-Rβ (flow cell 2): 2240 RU, murine PDGF-Rβ (flow cell 3): 2010 RU and human PDGF-sRα (flow cell 4): 1900 RU. All tested Z variants showed binding to both human and murine PDGF-Rβ, but no binding to human PDGF-Rα. The result is displayed in Figure 2. Z01982 and Z01977 showed the slowest dissociation curves to human PDGF-Rβ. Z01982 also showed the slowest dissociation curve to murine PDGF-Rβ.

Epitope test: The binding of human PDGF-Rβ to its natural ligand PDGF-BB in the presence of monomeric Z variants was tested. The ligand immobilization levels on the surfaces were: flow cell 2 (PDGF-BB): 1130 RU and flow cell 3 (PDGF-BB): 5090 RU. All three tested Z variants, Z01977, Z01980 and Z01982, blocked the PDGF-Rβ-PDGF-BB binding partially at a 1:1 molar ratio (i.e. 100 nM Z variant and 100 nM PDGF-Rβ). The result is displayed in Figure 3. When a ten times excess of Z variant compared to PDGF-Rβ was used, the blocking effect was almost complete. For the Aβ-binding Z variant used as negative control, no blocking effect was seen Dot blot analysis: A specificity test was made by adding purified dimeric Z variants to nitrocellulose membranes with 0.1 µg dots of different proteins. Bound Z variants were detected with chemiluminescence reacting HRP-conjugated antibodies. Seven binders were analyzed: Z01976, Z01977, Z01980, Z01982, Z01983, Z01994 and Z01995. The strongest signals were achieved for human and murine PDGF-Rβ for all tested Z variants (data not shown). Some background signals were seen for all Z variants, particularly to the immunoglobulins.

In summary, binding to both human and murine PDGF-Rβ, which are 79 % identical on an amino acid level, was exhibited by the Z variants in binding analyses and in dot blot. When assayed against PDGF-Rα, Fc and serum proteins in a Biacore instrument and in dot blot, the binders were shown to be negative, i.e. their binding was satisfactory concerning specificity with respect to PDGF-Rβ. Three of the binders (Z01977, Z01980 and Z01982) were shown to block binding of the receptor PDGF-Rβ to its natural ligand PDGF-BB in a Biacore assay. The result suggests that the three binders share an epitope on the receptor, as they showed the same blocking result. It is likely that the non-tested binders in the sequence cluster would show the same type of binding pattern.

### Example 3: Design and construction of a maturated library of PDGF-Rβ-binding Z variants

In this Example, a maturated library was constructed. The library was used for selections of PDGF-Rβ-binding polypeptides. Selections from maturated libraries are usually expected to result in binders with increased affinity (Orlova et al, Cancer Res 66(8):4339-48 (2006)).

### Materials and methods

Library design: The library was based on the sequences of the PDGF-Rβ-binding Z variants described in Examples 1 and 2. In the new library, 13 variable positions in the Z molecule scaffold were biased towards certain amino acid residues, according to a strategy based on the Z variant sequences defined in SEQ ID NO: 1-11. A degenerated 129 bp oligonucleotide encoding the possible variants was obtained from Scandinavian Gene Synthesis AB and denoted AFFI-1011. The theoretical frequencies (in %) and distributions of amino acid residues in the new library for the 13 variable Z positions are given in Table 2:

| **Possible amino acids** | **Amino acid positions in the Z variant molecule** | | | | | | | | | | | | |
|---|---|---|---|---|---|---|---|---|---|---|---|---|---|
| | Pos 2 | Pos3 | Pos 4 | Pos 6 | Pos7 | Pos 10 | Pos 11 | Pos 17 | Pos 18 | Pos 20 | Pos 21 | Pos 25 | Pos 28 |
| Ala (A) | 0 | 0 | 6.25 | 100 | 6.25 | 25 | 17 | 0 | 6.25 | 0 | 0 | 0 | 0 |
| Arg (R) | 50 | 17 | 9.375 | 0 | 9.375 | 0 | 8.25 | 100 | 9.375 | 45 | 0 | 37.5 | 17 |
| Asn (N) | 0 | 0 | 3.125 | 0 | 3.125 | 12.5 | 8.25 | 0 | 3.125 | 0 | 100 | 12.5 | 0 |
| Asp (D) | 0 | 0 | 3.125 | 0 | 3.125 | 12.5 | 8.25 | 0 | 3.125 | 0 | 0 | 0 | 0 |
| Cys(C) | 0 | 0 | 3.125 | 0 | 3.125 | 0 | 0 | 0 | 3.125 | 0 | 0 | 0 | 0 |
| Gln (Q) | 0 | 0 | 3.125 | 0 | 3.125 | 0 | 0 | 0 | 3.125 | 0 | 0 | 12.5 | 0 |
| Glu (E) | 0 | 0 | 3.125 | 0 | 3.125 | 12.5 | 8.25 | 0 | 3.125 | 0 | 0 | 0 | 0 |
| Gly (G) | 0 | 16.5 | 6.25 | 0 | 6.25 | 0 | 16.5 | 0 | 6.25 | 0 | 0 | 0 | 16.5 |
| His (H) | 0 | 0 | 3.125 | 0 | 3.125 | 0 | 0 | 0 | 3.125 | 0 | 0 | 12.5 | 0 |
| Ile (I) | 0 | 16.5 | 3.125 | 0 | 3.125 | 0 | 0 | 0 | 3.125 | 0 | 0 | 0 | 16.5 |
| Leu (L) | 50 | 17 | 9.375 | 0 | 9.375 | 0 | 0 | 0 | 9.375 | 0 | 0 | 0 | 17 |
| Lys (K) | 0 | 0 | 3.125 | 0 | 3.125 | 12.5 | 8.25 | 0 | 3.125 | 5 | 0 | 12.5 | 0 |
| Met (M) | 0 | 0 | 3.125 | 0 | 3.125 | 0 | 0 | 0 | 3.125 | 0 | 0 | 0 | 0 |
| Phe (F) | 0 | 0 | 3.125 | 0 | 3:125 | 0 | 0 | 0 | 3.125 | 0 | 0 | 0 | 0 |
| Pro (P) | 0 | 0 | 6.25 | 0 | 6.25 | 0 | 0 | 0 | 6.25 | 0 | 0 | 0 | 0 |
| Ser (S) | 0 | 16.5 | 9.375 | 0 | 9.375 | 0 | 8.25 | 0 | 9.375 | 0 | 0 | 12.5 | 16.5 |
| Thr (T) | 0 | 0 | 6.25 | 0 | 6.25 | 25 | 17 | 0 | 6.25 | 0 | 0 | 0 | 0 |
| Trp (W) | 0 | 0 | 3.125 | 0 | 3.125 | 0 | 0 | 0 | 3.125 | 45 | 0 | 0 | 0 |
| Tyr (Y) | 0 | 0 | 3.125 | 0 | 3.125 | 0 | 0 | 0 | 3.125 | 0 | 0 | 0 | 0 |
| Val (V) | 0 | 16.5 | 6.25 | 0 | 6.25 | 0 | 0 | 0 | 6.25 | 0 | 0 | 0 | 16.5 |
| Amber stop (q) | 0 | 0 | 3.125 | 0 | 3.125 | 0 | 0 | 0 | 3.125 | 5 | 0 | 0 | 0 |
| Stop (.) | 0 | 0 | 0 | 0 | 0 | 0 | 0 | 0 | 0 | 0 | 0 | 0 | 0 |
| # of aa | 2 | 6 | 20 | 1 | 20 | 6 | 9 | 1 | 20 | 4 | 1 | 6 | 6 |

Library construction: The degenerated oligonucleotide AFFI-1011 (5'-ctcgaggtagacaacaaattcaacaaagaackkvkynnkgcggctnnkgagatcrmmrvsttacctaacttaaaccgtnnkc aawrgaacgccttcatcmrwagtttavktgatgacccaagccaaagc) was amplified by PCR using 100 fmol AFFI-1011, 50 pmol forward primer AFFI-47 (5'-cccccccccctcgaggtagacaacaaattcaa) and 50 pmol reverse primer AFFI-50 (5'-cccccctgctagcaagttagcgctttggcttgggtcatc). An explanation of the nomenclature of degenerated nucleotides can be found in e.g. Biochemical Nomenclature and Related Documents, Portland Press, 1992. 95 PCR reactions were performed using AmpliTaq Gold polymerase (Applied Biosystems #N808-0244) in 10 cycles, each cycle consisting of one 15 s denaturation period at 96 °C, one 15 s annealing period at 60 °C and one 1 min extension period at 72 °C. After the amplification, the PCR products were pooled and purified using QIAquick® PCR purification kit (Qiagen). Fragment concentration and quality were determined by absorbance measurement and by analysis on an agarose gel. The amplified fragment was restriction cleaved at a concentration of 14 ng/µl using 1000 U of the enzymes *Xhol* and *Nhel* (New England Biolabs, #R0146L, #0131M). The 1500 µl reaction mixture was incubated at 37 °C for 3.5 hours. The cleaved fragments were purified using a QIAquick® PCR purification kit. Fragment concentrations were determined by absorbance measurements, and fragment quality by agarose gel analysis.

The phagemid vector pAY00065 was restricted with the same enzymes, purified and ligated with the amplified fragments. The amplified fragments (1.93 µg) and the vector (12 µg), in a molar ratio of 5:1, were ligated for 2 hours at RT. 50 U of T4 DNA ligase (Fermentas #EL0012) was used per µg of DNA in a ligation mixture of 6000 µl, and aliquoted into eppendorf tubes with 500 µl in each. The ligase was inactivated at 65 °C for 10 minutes and DNA was recovered by phenol/chloroform (Invitrogen) extraction and ethanol precipitation, followed by dissolution in sterile deionized water.

The ligation reactions (2 µl of approximately 150 ng/µl) were transformed into electrocompetent *E*. *coli* RRIΔM15 cells (100 µl). Immediately after electroporation, approximately 1 ml of SOC medium (TSB-YE media, 1 % glucose, 50 µM MgCl₂, 50 µM MgSO₄, 50 µM Nacl and 12.5 µM KCI) was added. The transformed cells were incubated at 37 °C for 40-50 min. Samples were taken for titration and for determination of the number of transformants. The cells were thereafter divided into 6 fractions which were inoculated in 1000 ml TSB-YE medium, supplemented with 2 % glucose and 100 µg/ml ampicillin, and cultivated overnight at 37 °C. The cells were pelleted for 8 min at 6000 g, re-suspended in a PBS/glycerol solution (approximately 20 % glycerol). The different cell fractions were aliquoted and stored at -80 °C.

Preparation of phage stock: Cells from the glycerol stock containing the phagemid vector were inoculated in 2 x 1000 ml TSB-YE medium, supplemented with 2 % glucose and 100 µg/ml ampicillin, and grown at 37 °C. When the cells reached an optical density (OD) of 0.65, the same amount of cells as initially inoculated was infected using a 10x molar excess of M13K07 helper phage (New England Biolabs). The cells were incubated for 30 minutes, centrifuged at 2000 *g* for 10 min and re-suspended. Subsequently, the cells were cultivated in 2x 1000 ml TSB-YE medium, supplemented with 100 µM IPTG for induction of expression, 25 µg/ml kanamycin and 100 µg/ml ampicillin, and grown overnight at 30 °C. The induced culture was harvested by centrifugation at 2500 *g* for 10 min. In order to separate the phage particles from the cells, the supernatant was precipitated in PEG/Nacl. The precipitation buffer was added to the supernatant in a 1:4 volume ratio and the resulting mixture was incubated on ice for 1 hour. Precipitated phages were pelleted by centrifugation at 10500 *g* at 4 °C for 30 min and resuspended in sterile H₂O. The precipitation procedure was repeated once and the phages were re-suspended in 1 ml PBS. The resulting phage solution was clarified from cells and cell debris by centrifugation, followed by filtration through a 0.45 µm filter. Glycerol was added to a final concentration of approximately 40 %. Phage stocks were stored at -80 °C.

Sequencing: Clones from the phage stock library of Z variants were sequenced in order to verify the content and to evaluate the outcome of the constructed library in relation to the library design. Sequencing was performed as described in Example 1. The amino acid distribution was calculated.

### Results

Library construction: The new library was designed based on a set of PDGF-Rβ-binding Z variants with verified binding properties (Example 1 and 2). The theoretical size of the designed library was 7.5 x 10⁸ Z variants. The actual size of the library, determined by titration and after transformation to *E*. *coli.* RRIΔM15 cells, was 5.3 x 10⁹ transformants.

The library quality was tested by sequencing of 95 transformants and by comparing their actual sequences with the theoretical design. The contents of the actual library compared to the designed library were shown to be satisfying. The locked positions in the designed amino acid sequence were reflected in the actual sequence in that only the expected amino acids occurred in these positions. Similarly, the biased or doped positions in the design were also reflected in the actual sequence in that most of the expected amino acids occurred in these positions.

A maturated library of PDGF-Rβ-binding polypeptides was thus successfully constructed.

### Example 4: Selection, screening and characterization of Z variants from a maturated library

### Materials and methods

The target protein, human recombinant PDGF-Rβ, was biotinylated as described in Example 1. Biotinylated dimeric Z00000 was coupled to streptavidin beads as described in Example 1.

Phage display selection of PDGF-Rβ-binding polypeptides: Phage display selections were performed against PDGF-Rβ essentially as described in Example 1 using the new library of Z variant molecules described in Example 3. Selection was initially performed in four tracks. These were further divided in two cycles, resulting in totally six tracks. Selection was performed in four cycles. See Figure 4B for an overview of target concentrations and number of washes. In order to reduce the number of background binders, phages were pre-incubated with Z00000-coupled streptavidin beads coated with Fc protein for 1 hour at RT prior to selection cycles 1-3.

Thereafter, non-biotinylated (1a, 1b and 2) and biotinylated human PDGF-Rβ (3a, 3b and 4) was added to the phage solution. Selection took place in blocked tubes (pre-blocked in selection buffer) incubated under agitation for two hours. To capture the phage-protein complexes, the selection solution was incubated with pre-blocked Z00000-coupled streptavidin beads (1a, 1 b and 2) or streptavidin beads (3a, 3b and 4) for 20 min and for 15 min, respectively. Thereafter, unbound phage particles were removed by washing with PBS-T 0.1. The number of washes was increased for each selection cycle, starting with 4 washes in the first cycle and ending with up to 20 washes in the last cycle. Washing was performed at RT (2 and 4), 37 °C (1 b and 3b) and 45 °C (1 a and 3a). The wash length was less than 1 minute in all steps, except for the last wash step in the last cycle which lasted 30 minutes. Bound phages were eluted using a low pH strategy where 500 µl of 50 mM glycine-HCl, pH 2.2, was added to the streptavidin beads. After 10 min incubation at RT, the solution was neutralized by addition of 450 µl PBS and 50 µl 1 M Tris-HCl, pH 8.

Amplification of phage particles: After each selection cycle, approximately 950 µl of the eluted phages from the different selection tracks were used to infect log phase *E*. *coli* RR1ΔM15 cells. Bead-bound phage were similarly infected. After 20 min of incubation at 37 °C, the cells infected with eluted phage and bead-bound phage from the same selection were pooled (cycle 1-3) or kept separate (cycle 4). The infected cells were harvested by centrifugation. The pellet was dissolved in a small volume of TSB-YE and subsequently spread onto TYE plates. The plates were thereafter incubated overnight at 37 °C. In the final selection cycle, bacteria were diluted before spreading onto TYE plates in order to form single colonies to be used in ELISA screening.

The cells from the TYE plates were re-suspended in TSB medium. A part of the re-suspended cells were stored in glycerol at -20 °C. An amount of suspended cells corresponding to 100-1000 times the number of eluted phages was inoculated in TSB-YE medium, supplemented with 2 % glucose and 100 µg/ml ampicillin. The cells were grown to log phase at 37 °C. The same amount of cells as inoculated before were infected with 10x excess of M13K07 helper phages (New England Biolabs) during 30 min at 37 °C. The cells were pelleted by centrifugation and re-suspended in TSB-YE medium supplemented with 25 µg/ml kanamycin, 100 µg/ml ampicillin and 100 µM IPTG to induce the production of Z variant molecules. The cultivations were incubated overnight at 30 °C. The phage particles were harvested by centrifugation and precipitation as described earlier.

ELISA screening of Z variants: Z variant molecules were produced by inoculation of single colonies, prepared as described above, in TSB-YE medium as described in Example 1. Half area 96 well ELISA plates were prepared as described in the same Example. After removal of the blocking solution from the wells, 50 µl of the periplasmic solution containing Z variant molecules was added to each well. The plates were incubated at RT for 1.5 hours. The plates were washed four times with PBS-T 0.05. Human PDGF-Rβ, at a concentration of 4.5 µg/ml in 50 µl PBS-T 0.1, was added to the wells and the plates were incubated for 1.5 h at RT. After washing as described above, target bound to the Z variants was detected by addition of the antibody against Fc conjugated to HRP, diluted 1:4000 in PBS-T 0.1. This was followed by incubation for 1 h at RT. Thereafter, developing was performed as described earlier. Absorbance was measured at 450 nm in an ELISA spectrophotometer. All steps from blocking to reading were performed manually.

Sequencing of potential binders: From the ELISA screening, individual clones from the different selection tracks were picked for sequencing. Clones with absorbance values from at least twice the background absorbance to ten times the background absorbance were sequenced. Amplification of gene fragments and sequence analysis of gene fragments were performed as described in Example 1.

Biacore binding analysis: In order to rank the sequenced clones, periplasmic fractions containing ABD tagged PDGF-Rβ-binding Z variants, previously prepared for ELISA, were analyzed in a Biacore instrument (GE Healthcare). Human PDGF-Rβ and HSA were immobilized onto a Biacore CM5 chip; PDGF-Rβ in flow cell 2 and HSA in flow cell 3 and 4. One flow cell surface on the chip (flow cell 1) was activated and deactivated for use as blank during analyte injections. The immobilization was performed according to the manufacturer's protocol. The periplasmic fractions were clarified at 16000 *g* for 5 min and injected at RT. In addition, some binders were analyzed at 37 °C. The running buffer was HBS-EP and the analytes were injected at a flow-rate of 10 µl/min for 5 minutes. After 5 minutes of dissociation, the surfaces were regenerated with one injection of 0.05 % SDS and one injection of 15 mM HCl. The results were analyzed using BiaEvaluation software. Sensorgrams from the blank surface (flow cell 1) were subtracted from the sensorgrams from the ligand surfaces.

Subcloning of Z variant molecules into expression vectors: Based on sequence analysis and binding analysis in Biacore, a number of clones were selected for subcloning into the expression vector pAY01449 as described in Example 1. Monomer and dimer Z variant fragments were amplified from the pAffi1/pAY00065 vectors as described earlier in Example 1.

Accl-digested pAY01449 vector and amplified Z variant fragments were ligated using T4 DNA ligase. The ligation mix was electroporated into electrocompetent *E*. *coli* TOP-10 cells. The resulting transformants were plated on TBAB plates, supplemented with 50 µg/ml of kanamycin, followed by incubation at 37 °C overnight. Thereafter, colonies with the different Z variant molecules were screened using PCR and the lengths of the PCR fragments were verified on agarose gels.

To verify the sequences, sequencing was performed with BigDye® Terminator v3.1 Cycle Sequencing Kit using biotinylated primers in separate reactions. The sequencing products were purified using a Magnatrix 8000 with streptavidin coated magnetic beads, and analyzed on an ABI PRISM® 3100 Genetic Analyser. The sequences were evaluated with Sequencher™ software.

Plasmid DNA was prepared from clones with confirmed sequence. The bacteria were grown overnight in TSB medium, supplemented with 50 µg/ml of kanamycin. The cells were pelleted by centrifugation. Plasmids were prepared using QlAprep Spin Miniprep Kit, and electroporated into electrocompetent *E. coli* BL21 (DE3) cells. The new plasmids were deposited at -80 °C. Aliquots of the TOP-10 and BL21 DE3 cultures were similarly stored in glycerol at -80 °C.

### Results

Phage display selection of PDGF-Rβ-binding polypeptides: Selection was performed in totally six parallel tracks with four cycles each. The different selection tracks differed in target concentration and wash conditions as follows: 1a) non-biotinylated target, high concentration, 37/45 °C wash, 1b) non-biotinylated target, low concentration, 37 °C wash, 2) non-biotinylated target, low concentration, RT wash, 3a) biotinylated target, high concentration, 37/45 °C wash, 3b) biotinylated target, low concentration, 7°C wash, and 4) biotinylated target, low concentration, RT wash. For each selection cycle, the target concentration was decreased and the washing conditions were more stringent. A pressure for structural stability was introduced in the selections by the high temperature wash (37 °C and 45 °C) of phage-target complexes. Phage particle titers and phage particle yield (% phage out/phage in) were determined after each cycle. For each selection cycle, the phage particle yield was higher (not shown), which indicated an enrichment of target binding clones.

ELISA screening of Z variants: Clones obtained after four selection cycles were produced in 96-well plates and screened for PDGF-Rβ-binding activity using ELISA. In total, 2x93 clones from the group of eluted phage and 2x93 clones from the group of bead-bound phage were analyzed. Many clearly positive clones were found with signals of up to 2.2 absorbance units (AU). Clones from all selection tracks (and from both eluted and bead-bound phage) showed positive signals. The negative controls (lysates from a negative clone, basically pAffil/pAY00065, without Z variant insert) were clearly negative (absorbance < 0.2 AU). The positive control (Z01977 from Example 1) gave an absorbance signal (0.5-1.4 AU) which was lower than most of the positive clones.

Sequencing: 192 clones with positive ELISA signals were sequenced (96 clones from the group "eluted phage" and 96 clones from the group "bead-bound phage"). Each individual Z variant was given an identification number, Z#####, as described earlier. In total, 163 new Z variant molecules were identified. The amino acid sequences of the 58 amino acid residues long Z variants are listed in Figure 1 and in the sequence listing as SEQ ID NO:370-532. The deduced PDGF-Rβ-binding motifs of these Z variants are listed in Figure 1 and in the sequence listing as SEQ ID NO:12-174. The amino acid sequences of the 49 amino acid residues long polypeptides predicted to constitute the complete three-helix bundle within each of these Z variants are listed in Figure 1 and in the sequence listing as SEQ ID NO:191-353. Among the sequenced clones, fifteen sequences occurred twice and one sequence occurred three times.

Clustering of the sequenced PDGF-Rβ-binding Z variants showed that the variants had similar sequences with an internal dissimilarity of down to 2 %, corresponding to a difference in one single amino acid.

Biacore binding analysis: Due to the large number of different Z variants with strong signals in ELISA, a second screening was performed using Biacore binding analysis. Clones with high ELISA signals, frequent occurrence (>1), origin in selections with washes at 37/45 °C and sequences similar to Z01982 (a sequence variant with high affinity fro PDGF-Rβ and Z01978 (a sequence variant with high Tm), were chosen for binding analysis. In total, 45 clones were chosen. Z01977 and Z01982 were included for comparison. A Z variant molecule binding to CD22 was used as negative control. The response levels acquired from the RT experiment against HSA were used for approximation of the relative concentrations. Apart from the response differences due to varying levels of Z variant molecule expression, the shapes of the sensorgrams in the RT experiment for the clones screened against PDGF-Rβ were similar. However, small dissimilarities were seen, and by relating the Z variant molecule expression levels to the sensorgrams from the PDGF-Rβ surface, the Z variants could be graded. In the experiment performed at 37 °C, small distinctions between the Z variants could be seen by relating the responses against PDGF-Rβ with the responses against HSA. Thus, five Z variants (Z02465, Z02477, Z02483, Z02516 and Z02558) were picked for subcloning and further analysis, based on their low response to HSA and high response to PDGF-Rβ, their slow dissociation curve, their occurrence in duplicate in the selection, and/or their strong binding at 37 °C.

Subcloning of Z variant molecules into expression vectors: Five clones, Z02465, Z02477, Z02483, Z05.16 and Z02558, were successfully subcloned into the expression vector pAY01449 as monomers and dimers as described previously in this Example.

### Example 5: Production and characterization of a subset of PDGF-Rβ-binding Z variants

### Materials and methods

### Protein expression and purification: Transformed E. coli BL21 (DE3) cultures from the subcloning described in Example 4 were grown at 37 °C to an optical density of approximately 1 (diluted 10x), and protein expression was induced by addition of 1 M IPTG (0.5 ml/l culture). Cultures were harvested by centrifugation (20 min, 15900 g) 5 h after induction. The supernatants were discarded and the cell pellets were collected and stored at -20 °C. Expression levels of soluble and unsoluble proteins were analyzed using SDS-PAGE by ocular determination of Coomassie-stained gels.

PDGF-Rβ-binding Z variants constituting monomers and dimers of Z02465, Z02477, Z02483, Z0516 and Z02558 (in His₆-Z#####-Cys form and in His₆-(Z#####)₂-Cys form; i.e. expressed as the constructs MGSSHHHHHHLQ-[Z#####]-VDC and MGSSHHHHHHLQ-[Z#####][Z#####]-VDC, respectively) were purified from cell pellets under denatured conditions on Ni-NTA Superflow Columns (Qiagen), buffer exchanged to PBS using PD-10 columns (GE Healthcare), aliquoted and stored at -80 °C.

Protein characterization was performed essentially as in Example 2. CD analysis: The purified monomeric Z variants were thawed and diluted to 0.5 mg/ml in PBS. For each diluted Z variant, a CD spectrum at 250-195 nm was obtained at 20 °C, followed by a variable temperature measurement (VTM) to determine the melting temperature (Tm). After incubation at 20 °C, a second CD spectrum 250-195 nm was obtained at 20 °C to verify that the Z variant molecule retained its a-helical structure after the VTM. In the VTM, the absorbance was measured at 221 nm while the temperature was raised from 20 to 90 °C, with a temperature slope of 5 °C/min. The CD measurements were performed on a Jasco J-810 spectropolarimeter using a cell with an optical path-length of 1 mm.

Biacore binding and kinetic analysis: Five monomeric Z variant molecules, with NEM-blocked (N-ethylmaleimide, Pierce #23030) cysteines and with His₆-tags (His₆-Z#####-Cys-NEM), were subjected to interaction studies in a Biacore instrument (GE Healthcare) with human PDGF-Rβ, murine PDGF-Rβ and human PDGF-Rα. The three PDGF receptors had previously (Example 2) been immobilized onto different flow cells of a CM5 chip. The analytes were diluted in the running buffer HBS-EP to a final concentration of 100 nM, injected in duplicates or triplicates at a flow-rate of 20 µl/minute during 3 minutes. After 3 minutes of dissociation, the surfaces were regenerated with one injection of 10 mM HCl.

The binding affinities of Z variants Z02465 and Z02483 (in His₆-Z#####-Cys-NEM form) and the first generation binder Z01982 (in His₆-Z##### form) for human and murine PDGF-Rβ were determined in Biacore. Human and murine PDGF-Rβ were immobilized in different flow cells of a Biacore CM5 chip as described earlier. Different concentrations of the Z variants (100 nM, 40 nM, 16 nM, 6.4 nM and 2.56 nM) in HBS-EP buffer were injected at a flow-rate of 50 µl/minute for 1 minute. After 1 minute of dissociation, the surfaces were regenerated with one injection of 0.05 % SDS and one injection of 10 mM HCl.

One flow cell surface of the chip was activated and deactivated for use as blank during analyte injections. Sensorgrams from the blank surface were subtracted from the sensorgrams from the ligand surfaces. BiaEvaluation software (GE Healthcare) was used for analyzing the results and calculating the dissociation constants (K_{D}).

Dot blot analysis: Three PDGF-Rβ-binding Z variants were tested for specificity by dot blot analysis performed as described in Example 2 using dimeric Z variant molecules with N terminal His₆ tags and C terminal cysteines.

Flow cytometry: Five different dimeric Z variant molecules (20 or 5 µg/ml in PBS), were added to 0.5 x 10⁶ cells PDGF-Rβ expressing AU565 (human mammary gland carcinoma cells, ATCC) in a 5 ml Falcon tube. The mixtures were incubated for 1 hour on ice. Subsequently, the cells were washed in PBS and pelleted in a centrifuge at 200 *g* for 3 minutes. The bound Z variant molecules were detected by addition of goat antibody against Z variants, followed by Alexa Fluor® 647 conjugated anti-goat lg (Invitrogen #A20347, 5 µg/ml). After each addition, the cells were incubated for 45-60 minutes and thereafter washed twice in PBS. As a control, AU565-cells were stained with a commercially available goat anti-PDGF-Rβ antibody (R&D systems) followed by an Alexa Fluor® 647 conjugated anti-goat lg antibody (Invitrogen). After staining, cells were washed, re-suspended and analyzed using a FACSCanto II flow cytometer (BD Biosciences). In a second set of flow cytometry analysis, Alexa Fluor® 647-conjugated His₆-(Z02465)₂-Cys, His₆-(Z02483)₂-Cys, and His₆-(Z02516)₂-Cys were used for staining. The Z variants were conjugated using Alexa Fluor® 647 C2-maleimid reagent (Invitrogen). AU565 cells, 0.5 x 10⁶ per tube (5 ml Falcon tube) were mixed with a 3-fold dilution series of the three conjugated binders. After one hour of incubation on ice, cells were washed as described above and resuspended in PBS for Flow Cytometry analysis using FACSCanto II.

Immunofluorescence: AU565 cells were grown overnight on multiwell slides (Histolab) to yield a monolayer of cells. The next day, cells were rinsed with PBS and subjected to staining. The cells were stained with any one of three Z variant molecules (10 µg/ml) or a goat anti-PDGF-Rβ antibody (5 µg/ml), diluted in PBS on unfixed cells. After one hour of incubation, the wells were washed. The Z variant molecules were detected with a purified goat anti-Z Ig (5 µg/ml), followed by anti-goat IgG Alexa488 (5 µg/ml, Invitrogen #A21467). The goat anti-PDGF-Rβ antibody was detected by anti-goat IgG Alexa488. After each antibody addition, the wells were incubated for 1 hour and washed with PBS. The cells were fixed for 10 minutes with 3% formaldehyde in PBS, followed by wash with PBS. The wells were dried and the glass slides were mounted with anti-fading solution (Vectashield mounting medium for fluorescence with DAPI, Vector Laboratories #H-1200), and analyzed using a Leica DM-LA microscope (Leica Microsystems) equipped with a live imaging video camera.

### Results

Protein expression and purification: All expressed variants, Z02465, Z02477, Z02483, Z02516 and Z02558 (in His₆-Z#####-Cys and in His₆-(Z#####)₂-Cys form) gave good yields of soluble gene product. The amount of IMAC-purified Z variant molecules ranged from 5.5 mg to 12.1 mg per purified batch and the *in vitro* solubility for all variants were good. The purity was estimated on SDS-PAGE to exceed 90 % for all variants. The correct molecular weights were verified with LC-MS.

CD analysis: CD spectrum measurements were performed at 20 °C. At that temperature, the a-helical structures of the Z variants were almost fully developed. An overlay of the spectrums obtained after the variable temperature measurements (heating to 90 °C followed by cooling to 20 °C) on the spectrums obtained before the variable temperature measurement showed that all Z variants fold back to their a-helical structures after heating to 90 °C (result not shown). The melting temperatures were determined from the variable temperature measurements and are shown in Table 3.

**Table 3. Melting temperatures of Z variants.**

| **Z variant molecule** | **Tm (°C)** |
|---|---|
| His₆-Z02465-Cys | 46 |
| His₆-ZO2477-Cys | 46 |
| His₆-ZO2483-Cys | 44 |
| His₆-Z02516-Cys | 42 |
| His₆-Z02558-Cys | 39 |
| His₆-(Z02477)₂-Cys | 46 |

Biacore binding and kinetic analysis: The binding of five Z variant molecules (in His₆-Z##### Cys-NEM form) to human and murine PDGF-Rβ as well as to human PDGF-Rα was tested in Biacore by injecting each of the monomeric Z variant molecules Z02465, Z02477, Z02483, Z02516 and Z02558 over surfaces containing the three receptors. Comparisons were made with two variants from the first selection (Example 1). Z01977 and Z01982 (in His₆-Z##### form). All tested Z variants showed binding to both human and murine PDGF-Rβ but no binding to human PDGF-Rα. Z02465 and Z02483 showed the highest response curves against both the human and the murine receptor. The results are displayed in Figure 5.

The binding affinities of two maturated Z variants (Z02465 and Z02483) and one Z variant from the first selection (Z01982; Example 1), were determined by calculation of the dissociation constant K_{D}. The calculation was based on the results from a Biacore experiment where the Z variant molecules, in five different concentrations, were run over a Biacore chip containing human and murine PDGF-Rβ. The ligand immobilisation grades on the surfaces were: flow cell 2 (hPDGF-Rβ): 1110 RU, flow cell 3 (hPDGF-Rβ): 3010 RU and flow cell 4 (mPDGF-Rβ): 3080 RU.

The calculations of K_{D} were performed using a Langmuir 1:1 binding model on sensorgrams from the concentration series against the human (flow cell 2) and the murine (flow cell 4) receptor. Good curve fitting was achieved for Z01982 (all five concentrations) as well as for Z02465 and Z02483 (all except one concentration) against human PDGF-Rβ (Figure 6). For the murine receptor, all Z variant concentrations were used in the calculations (sensorgrams not shown). The calculated dissociation constants are shown in Table 4 below.

**Table 4. Dissociation constants (K_{D}) for PDGF-Rβ-binding Z variants**

| **Z variant** | **human PDGF-Rβ** | **murine PDGF-Rβ** |
|---|---|---|
| His₆-Z01982 | 4 nM | 27 nM |
| His₆-Z02465-Cys-NEM | 500 pM | 7 nM |
| His₆-Z02483-Cys-NEM | 400 pM | 6 nM |

Dot blot analysis: The specificities of Z02516, Z02483 and Z02465 (in His₆-(Z#####)₂-Cys form) for PDGF-Rβ were tested using dot blot analysis. 22 different proteins were blotted onto nitrocellulose membranes. The 22 proteins included 16 high abundant human serum proteins, PDGF-Rβ and recombinant human and mouse PDGF-Rβ. The three Z variant molecules bound to human and mouse PDGF-Rβ, but not to PDGF-Rα or to any of the other 19 molecules. Thus, the specificity of the tested Z variants was satisfying (data not shown).

Flow cytometry: The cell binding capacity of PDGF-Rβ-binding Z variant molecules were analyzed using flow cytometry. PDGF-Rβ expressing AU565 cells were stained with 20 µg/ml of Z02465, Z02483, Z02477, Z02516 or Z02558 (in His₆-(Z#####)₂-Cys form). The Z variant molecules were detected with a goat anti-Z antibody and Alexa Fluor® 647 conjugated anti-goat IgG. A goat anti-PDGF-Rβ antibody was used as a control. All five Z variant molecules gave shifts in fluorescence intensity (data not shown).

In addition, the three Z variants molecules Z02465, Z02483 and Z02516 (in His₆-(Z#####)₂-Cys form) were conjugated with Alexa Fluor® 647 on the C-terminal cysteine and their abilities to bind to AU565 cells were compared. The cells were incubated with the molecules in a 3-fold titration series, ranging between 20000 to 1 ng/ml. The three Z variant molecules showed an equivalent binding to AU565 cells. The result is displayed in Figure 8.

Immunofluorescence: PDGF-Rβ, expressed on the membranes of AU565 cells, was detected by immunofluorescence staining using any one of the dimeric Z variants Z02465, Z02483 and Z02516 (in His₆-(Z#####)₂-Cys form) or a goat anti-PDGF-Rβ antibody. Membrane staining was observed with the control antibody as well as with the three Z variants (Figure 7). Staining with Z02483 resulted in a spotted staining pattern in addition to membranous staining which could be due to internalized PDGF-Rβ.

In conclusion, binding to both human and murine PDGF-Rβ was shown by the six investigated Z variants in a Biacore instrument and in dot blot analysis. When assayed against PDGF-Rα, Fc and serum proteins (Biacore and dot blot), the Z variants were shown to be negative, i.e. the Z variants showed specificity for PDGF-Rβ. In flow cytometry experiments, five dimeric Z variants showed binding to PDGF-Rβ-expressing cells. The microscopic analysis showed that the three strongest Z variants bound to the membrane of cells, reminiscent of the PDGF-Rβ specific control antibody. The variant Z02483 also bound to other structures, which most likely were internal vesicles. This suggests that PDGF-Rβ resides inside the cell. The melting temperatures for the maturated Z variants were improved compared to the Z variants from the first library selection. The variants Z02465 and Z02477 reached a Tm of 46 °C.

### Example 6: Characterization and binding analyses of mutated Z variants

Two Z variants from the selection described in Example 4 were subjected to site-directed mutagenesis in order to create new PDGF-Rβ-binding polypeptides.

### Materials and methods

The PDGF-Rβ binding motifs of Z variants Z02465 (SEQ ID NO:60) and Z02483 (SEQ ID NO:78) were mutated to create the new Z variants Z03358 (from Z02465, *PBM* listed as SEQ ID NO:179 and the sequences of the 49 and 58 amino acid residues long polypeptides listed as SEQ ID NO:358 and SEQ ID NO:537, respectively), Z02831, Z02832, Z02833 and Z02834 (all four from Z02483, *PBM*:s listed as SEQ ID NO:175-178 and the sequences of the 49 and 58 amino acid residues long polypeptides listed as SEQ ID NO:354-357 and SEQ ID NO:533-536, respectively).

New amino acids were introduced in relevant positions of the parent scaffold in different expression vectors. The constructs encoded by the expression vectors were MGSSLQ-[Z#####]-VDC (Z02465), M-[Z#####]-C (Z03358) and MGSSHHHHHHLQ-[Z#####]-VD (Z02831, Z02832, Z02833 and Z02834), each M being removed by the host cell during production. The Z variants were generated using oligonucleotides with varied codons and a PCR based mutagenesis technique. Obtained PCR fragments were ligated into a restriction enzyme cleaved expression vector, as described in Example 1 or by using In-Fusion technology (Clontech, #639607, and transformed into *E. coli* TOP 10 cells. Colonies were screened by PCR and sequences were verified essentially as described in Example 1. Plasmids were prepared and transformed into *E*. *coli* BL21 (DE3).

His₆-tagged proteins were expressed, released by sonication and purified essentially as described in Example 2. Proteins without His₆-tag were expressed and then purified as follows: *E*. *coli* cells harboring soluble Z02465-Cys or Z03358-Cys were suspended in 20 mM Tris-HCl, pH 7.1. To disrupt the cells and release the intracellular content, the cell suspensions were heated to 85 °C for 3 minutes. The lysates were clarified by centrifugation followed by filtration and loaded on 25 ml Q Sepharose FF (GE Healthcare) packed in an XK26 column (GE Healthcare), previously equilibrated with 20 mM Tris-HCl, pH 7.1. After column wash with 5 column volumes (CV) 20 mM Tris-HCl, pH 7.1, bound proteins were eluted with a linear gradient of 0-0.5 M NaCl in 20 mM Tris-HCl, pH 7.1, during 10 CV. The flow rate was 5 ml/min and the 280 nm signal was monitored. Fractions containing Z02465-Cys or Z03358-Cys were identified by SDS-PAGE analysis. Relevant fractions were pooled and pH was adjusted to 8.0 by addition of 1 M Tris-HCl, pH 8.0, to a final concentration of 50 mM.

The C-terminal cysteine on the constructs was reduced by addition of DTT to 20 mM, followed by incubation at 40 °C for 3 minutes. After reduction, acetonitrile (ACN) was added to a final concentration of 5 %, and reduced Z02465-Cys or Z03358-Cys was loaded on 1 ml Resource 15 RPC columns (GE Healthcare), previously equilibrated with RPC A-buffer (0.1 % TFA, 5 % ACN, 95 % water). After column wash with 10 CV RPC A-buffer, bound proteins were eluted with a linear gradient of 0-40 % RPC B-Buffer (0.1 % TFA, 80 % ACN, 20 % water). The flow rate was 1 ml/min and the 280 nm-signal was monitored. Fractions containing pure Z02465-Cys or Z03358-Cys were identified by SDS-PAGE analysis. Relevant fractions were pooled and the buffer was changed to 10 mM NH₄HC0₃, pH 8.0, using PD10 columns (GE Healthcare). The new Z variants were analyzed by LC-MS for verification of the molecular weights.

The purified Z variants were diluted to 0.2 or 0.5 mg/ml in PBS and CD analyses were performed between 250-195 nm at 20 °C. Subsequently, VTM:s were performed at 220 nm between 20-90 °C. Another CD spectrum was obtained for the Z variants after the VTM.

Binding of Z variants Z02831, Z02832, Z02833, Z02834 and Z02483 (parent molecule; protein produced in Example 4 and NEM treated as described in Example 5) to PDGF receptors (human and murine) was tested in a Biacore instrument. The receptors were immobilized in different flow cells on Biacore chips as described in Example 2, and the Z variants, diluted in HBS-EP buffer, were injected over the chip surface in different concentrations. The results were analyzed as described in Example 2. Kinetic analysis of Z variants Z02465 and Z03358 (NEM treated as described in Example 5) was performed essentially as described in Example 5, using the Z variant concentrations 4 nM, 20 nM and 100 nM and the flow rate 30 µl/min.

### Results

All variants were successfully expressed and showed satisfying purity. Their theoretical molecular weights were verified by LC-MS. The melting temperatures (Tm), an estimate of the molecular stability, were determined with CD analyses: Z02831 Tm = 44 °C, Z02832 Tm = 42 °C, Z02833 Tm = 49 °C, Z02834 Tm = 49 °C and Z003358 Tm = 42 °C. The Z variants regained their helical structure after the VTM, i.e. melting was reversible.

One of the mutated Z variants, Z02834, showed a faster off rate from human PDGF-Rβ than its parent molecule Z02483. The other Z02483 mutants (Z02831, Z02832 and Z02833) showed similar binding for human PDGF-Rβ as Z02483. All mutated Z variants showed better binding to human PDGF-Rβ than to murine PDGF-Rβ. The dissociation constants (K_{D}) were calculated as described in Example 5 using sensorgrams from the 4 nM and 20 nM injections. The calculated K_{D} values are shown in Table 5 below.

**Table 5. Dissociation constants (K_{D}) for PDGF-Rβ binding Z variants**

| **Z variant** | **human PDGF-Rβ** | **murine PDGF-Rβ** |
|---|---|---|
| Z02465-Cys-NEM | 640 pM | 7 nM |
| Z03358-Cys-NEM | 440 pM | 7 nM |

## Claims

1. Platelet derived growth factor receptor beta (PDGF-Rβ) binding polypeptide, comprising a platelet derived growth factor receptor beta binding motif, *PBM*, which motif consists of the amino acid sequence selected from
i) EX₂X₃X₄AAX₇EID X₁₁LPNLX₁₆X₁₇X₁₈QW NAFtX₂₅X₂₆LX₂₈X_{29,}
wherein, independently of each other,
X₂ is selected from L, R and I;
X₃ is selected from R, I, L, V, K, Q, S, H, and A;
X₄ is selected from A, R, N, D, Q, E, H, K, M, S, T, W, F and V;
X₇ is selected from A, R, D, Q, E, G, K and S;
X₁₁ is selected from A, R, N, D, E, G, K, S, T and Q;
X₁₆ is selected from N and T;
X₁₇ is selected from R and K;
X₁₈ is selected from A, R, N, D, C, Q, E, G, L, K, M, S, T, W and V;
X₂₅ is selected from K, R, Q, H, S, G and A;
X₂₆ is selected from S and K;
X₂₈ is selected from V, R, I, L and A;
X₂₉ is selected from D and K;
and
ii) an amino acid sequence which has at least 90 % identity to the sequence defined in i), and wherein
the PDGF-Rβ-binding polypeptide binds to PDGF-Rβ such that the K_{D} value of the interaction is at most 1 x 10⁻⁶ M.

2. PDGF-Rβ-binding polypeptide according to claim 1, wherein i) is a sequence selected from SEQ ID NO: 1-179.

3. PDGF-Rβ-binding polypeptide according to claim 2, wherein i) is selected from SEQ ID NO:2-3, SEQ ID NO:5, SEQ ID NO:7, SEQ ID NO:11-12, SEQ ID NO:18-19, SEQ ID NO:38, SEQ ID NO:42, SEQ ID NO:44, SEQ ID NO:47, SEQ ID NO:60-62, SEQ ID NO:64, SEQ ID NO:67-68, SEQ ID NO:71-72, SEQ ID NO:78, SEQ ID NO:80-81, SEQ ID NO:83, SEQ ID NO:86, SEQ ID NO:91-92, SEQ ID NO:94-97, SEQ ID NO: 101-103, SEQ ID NO:105, SEQ ID NO:109, SEQ ID NO:111, SEQ ID NO:116, SEQ ID NO:119, SEQ ID NO:133, SEQ ID NO:137, SEQ ID NO:139-140, SEQ ID NO:149, SEQ ID NO:153, SEQ ID NO:160, SEQ ID NO:164, SEQ ID NO:170, SEQ ID NO:174 and SEQ ID NO:179.

4. PDGF-Rβ-binding polypeptide according to any preceding claim, in which said PDGF-Rβ-binding motif forms part of a three-helix bundle protein domain.

5. PDGF-Rβ-binding polypeptide according to any preceding claim, which comprises an amino acid sequence selected from:
i) K-[*PBM*]-DPSQSXₐX_{b}LLX_{c}EAKKLNDX_{d}Q;
wherein
[*PBM*] is a PDGF-Rβ-binding motif as defined in any one of claims 1-3;
Xₐ is selected from A and S;
X_{b} is selected from N and E;
X_{c} is selected from A and S;
X_{d} is selected from A and S;
and
ii) an amino acid sequence which has at least 80 % identity to any one of the sequences defined above.

6. PDGF-Rβ-binding polypeptide according to claim 5, wherein i) is selected from SEQ ID NO:180-358.

7. PDGF-Rβ-binding polypeptide according to claim 6, wherein i) is selected from SEQ ID NO:181-182, SEQ ID NO:184, SEQ ID NO:186, SEQ ID NO: 190, SEQ ID NO:239, SEQ ID NO:251, SEQ ID NO:257, SEQ ID NO: 290, SEQ ID NO:332 and SEQ ID NO:358.

8. PDGF-Rβ-binding polypeptide according to any preceding claim, the amino acid sequence of which comprises a sequence selected from SEQ ID NO:359-537.

9. PDGF-Rβ-binding polypeptide according to claim 8, the amino acid sequence of which comprises a sequence selected from SEQ ID NO:360-361, SEQ ID NO:363, SEQ ID NO:365, SEQ ID NO:369, SEQ ID NO:418, SEQ ID NO:430, SEQ ID NO:436, SEQ ID NO:469, SEQ ID NO:511 and SEQ ID NO:537.

10. A polynucleotide encoding a polypeptide according to any preceding claim.

11. PDGF-Rβ-binding polypeptide according to any one of claims 1-9 for use as a medicament.

12. Combination of a PDGF-Rβ-binding polypeptide according to any one of claims 1-9 and a therapeutic agent.

13. Combination according to claim 12 for use as a medicament.

14. PDGF-Rβ-binding polypeptide or combination according to any one of claims 1-9 and 12, for treatment of a PDGF-Rβ-related condition selected from cancer disease, such as gliomas, sarcomas, and leukemias; vascular disorders, such as atherosclerosis, restenosis, pulmonary hypertension, and retinal diseases; fibrotic diseases, such as pulmonary fibrosis, liver cirrhosis, scleroderma, glomerulosclerosis, and cardiac fibrosis.

## Patentansprüche

1. Plättchen-abgeleitetes Wachstumsfaktor-Rezeptor beta-Bindungs polypeptid (PDGF-Rβ-Bindungspolypeptid), umfassend ein Plättchen-abgeleitetes Wachstumsfaktor-Rezeptor beta-Bindungsmotiv, PBM, welches Motiv aus der Aminosäuresequenz besteht, die ausgewählt ist aus
i) EX₂X₃X₄AAX₇EID X₁₁LPNLX₁₆X₁₇X₁₈QW NAFIX₂₅X₂₆LX₂₈X₂₉,
wobei, unabhängig voneinander,
X₂ aus L, R und I ausgewählt ist;
X₃ aus R, I, L, V, K, Q, S, H und A ausgewählt ist;
X₄ aus A, R, N, D, Q, E, H, K, M, S, T, W, F und V ausgewählt ist;
X₇ aus A, R, D, Q, E, G, K und S ausgewählt ist;
X₁₁ aus A, R, N, D, E, G, K, S, T und Q ausgewählt ist;
X₁₆ aus N und T ausgewählt ist;
X₁₇ aus R und K ausgewählt ist;
X₁₈ aus A, R, N, D, C, Q, E, G, L, K, M, S, T, W und V ausgewählt ist;
X₂₅ aus K, R, Q, H, S, G und A ausgewählt ist;
X₂₆ aus S und K ausgewählt ist;
X₂₈ aus V, R, I, L und A ausgewählt ist;
X₂₉ aus D und K ausgewählt ist;
und
ii) einer Aminosäuresequenz, die eine mindestens 90%ige Identität mit der in i) definierten Sequenz aufweist, und wobei
das PDGF-Rβ-Bindungspolypeptid an PDGF-Rβ dergestalt bindet, dass der K_{D}-Wert der Interaktion höchstens 1 x 10⁻⁶ M beträgt.

2. PDGF-Rβ-Bindungspolypeptid nach Anspruch 1, wobei i) eine aus SEQ ID NO:1-179 ausgewählte Sequenz ist.

3. PDGF-Rβ-Bindungspolypeptid nach Anspruch 2, wobei i) ausgewählt ist aus: S E Q ID NO:2-3, SEQ ID NO:5, SEQ ID NO:7, SEQ ID NO:11-12, SEQ ID NO-18-19, SEQ ID NO:38, SEQ ID NO:42 SEQ ID NO:44, SEQ ID NO:47 SEQ ID NO:60-62, SEQ ID NO:64, SEQ ID NO:67-68, SEQ ID NO:71-72, SEQ ID NO:78, SEQ ID NO:80-81, SEQ ID NO:83, SEQ ID NO:86, SEQ ID NO:91-92, SEQ ID NO:94-97, SEQ ID NO:101-103, SEQ ID NO:105, SEQ ID NO:109, SEQ ID NO:111, SEQ ID NO:116, SEQ ID NO:119, SEQ ID NO:133, SEQ ID NO:137, SEQ ID NO:139-140, SEQ ID NO:149, SEQ ID NO:153, SEQ ID NO:160, SEQ ID NO:164, SEQ ID NO:170, SEQ ID NO:174 und SEQ ID NO:179.

4. PDGF-Rβ-Bindungspolypeptid nach einem der vorangehenden Ansprüche, wobei das PDGF-Rβ-Bindungsmotiv einen Teil einer Drei-Helixbündel-Proteindomäne bildet.

5. PDGF-Rβ-Bindungspolypeptid nach einem der vorangehenden Ansprüche, das eine Aminosäuresequenz umfasst, ausgewählt aus:
i) K-[*PBM*]-DPSQSXₐX_{b}LLX_{c}EAKKLNDX_{d}Q;
wobei
[*PBM*] ein PDGF-Rβ-Bindungsmotiv wie nach einem der Ansprüche 1-3 definiert ist;
Xₐ aus A und S ausgewählt ist;
X_{b} aus N und E ausgewählt ist;
X_{c} aus A und S ausgewählt ist;
X_{d} aus A und S ausgewählt ist;
und
ii) einer Aminosäuresequenz, die eine mindestens 80%ige Identität mit einer der vorstehend definierten Sequenzen aufweist.

6. PDGF-Rβ-Bindungspolypeptid nach Anspruch 5, wobei i) aus SEQ ID NO:180-358 ausgewählt ist.

7. PDGF-Rβ-Bindungspolypeptid nach Anspruch 6, wobei i) ausgewählt ist aus: SEQ ID NO:181-182, SEQ ID NO:184, SEQ ID NO:186, SEQ ID NO:190, SEQ ID NO:239, S E Q ID NO:251, SEQ ID NO:257, SEQ ID NO:290, SEQ ID NO:332 und SEQ ID NO:358.

8. PDGF-Rβ-Bindungspolypeptid nach einem der vorangehenden Ansprüche, dessen Aminosäuresequenz eine aus SEQ ID NO:359-537 ausgewählte Sequenz umfasst.

9. PDGF-Rβ-Bindungspolypeptid nach Anspruch 8, dessen Aminosäuresequenz eine Sequenz umfasst, ausgewählt aus: SEQ ID NO:360-361, SEQ ID NO:363, SEQ ID NO:365, SEQ ID NO:369, SEQ ID NO:418 SEQ ID NO:430, SEQ ID NO:436, SEQ ID NO:469, SEQ ID NO:511 und SEQ ID NO:537.

10. Polynukleotid, kodierend für ein Polypeptid nach einem der vorangehenden Ansprüche.

11. PDGF-Rβ-Bindungspolypeptid nach einem der Ansprüche 1-9 zur Anwendung als ein Arzneimittel.

12. Kombination aus einem PDGF-Rβ-Bindungspolypeptid nach einem der Ansprüche 1-9 und einem therapeutischen Mittel.

13. Kombination nach Anspruch 12 zur Anwendung als ein Arzneimittel.

14. PDGF-Rβ-Bindungspolypeptid oder Kombination nach einem der Ansprüche 1-9 und 12 zur Behandlung eines PDGF-Rβ-bedingten Zustands, ausgewählt aus Krebserkrankungen, wie zum Beispiel Gliomen, Sarkomen und Leukämien; Gefäßerkrankungen, wie zum Beispiel Atherosklerose, Restenose, pulmonaler Hypertonie und Retinaerkrankungen; und fibrotischen Erkrankungen, wie zum Beispiel pulmonaler Fibrose, Leberzirrhose, Sklerodermie, Glomerulosklerose und kardialer Fibrose.

## Revendications

1. Polypeptide se liant au récepteur bêta du facteur de croissance dérivé des plaquettes (PDGF-Rβ), comprenant un motif de liaison au récepteur bêta du facteur de croissance dérivé des plaquettes, *PBM*, ledit motif étant constitué de la séquence d'acides aminés choisie parmi :
i) EX₂X₃X₄AAX₇EID X₁₁LPNLX₁₆X₁₇X₁₈QW NAFIX₂₅X₂₆LX₂₈X₂₉,
dans laquelle, de manière réciproquement indépendante :
X₂ est choisi parmi L, R et I ;
X₃ est choisi parmi R, I, L, V, K, Q, S, H et A ;
X₄ est choisi parmi A, R, N, D, Q, E, H, K, M, S, T, W, F et V ;
X₇ est choisi parmi A, R, D, Q, E, G, K et S ;
X₁₁ est choisi parmi A, R, N, D, E, G, K, S, T et Q ;
X₁₆ est choisi parmi N et T ;
X₁₇ est choisi parmi R et K ;
X₁₈ est choisi parmi A, R, N, D, C, Q, E, G, L, K, M, S, T, W et V ;
X₂₅ est choisi parmi K, R, Q, H, S, G et A ;
X₂₆ est choisi parmi S et K ;
X₂₈ est choisi parmi V, R, I, L et A ;
X₂₉ est choisi parmi D et K ;
et
ii) une séquence d'acides aminés qui possède une identité d'au moins 90 % à la séquence définie sous i), et polypeptide dans lequel
le polypeptide de liaison au PDGF-Rβ se lie au PDGF-Rβ de telle sorte que la valeur K_{D} de l'interaction s'élève au maximum à 1 x 10⁻⁶ M.

2. Polypeptide de liaison au PDGF-Rβ selon la revendication 1, dans lequel i) est une séquence choisie parmi les SEQ ID NO: 1 à 179.

3. Polypeptide de liaison au PDGF-Rβ selon la revendication 2, dans lequel i) est choisi parmi les SEQ ID NO: 2-3, la SEQ ID NO: 5, la SEQ ID NO: 7, les SEQ ID NO: 11-12, les SEQ ID NO: 18-19, la SEQ ID NO: 38, la SEQ ID NO: 42, la SEQ ID NO: 44, la SEQ ID NO: 47, les SEQ ID NO: 60-62, la SEQ ID NO: 64, les SEQ ID NO: 67-68, les SEQ ID NO: 71-72, la SEQ ID NO: 78, les SEQ ID NO: 80-81, la SEQ ID NO: 83, la SEQ ID NO: 86, les SEQ ID NO: 91-92, les SEQ ID NO: 94-97, les SEQ ID NO: 101-103, la SEQ ID NO: 105, la SEQ ID NO: 109, la SEQ ID NO: 111, la SEQ ID NO: 116, la SEQ ID NO: 119, la SEQ ID NO: 133, la SEQ ID NO: 137, les SEQ ID NO: 139-140, la SEQ ID NO: 149, la SEQ ID NO: 153, la SEQ ID NO: 160, la SEQ ID NO: 164, la SEQ ID NO: 170, la SEQ ID NO: 174 et la SEQ ID NO: 179.

4. Polypeptide de liaison au PDGF-Rβ selon l'une quelconque des revendications précédentes, dans lequel ledit motif de liaison au PDGF-Rβ fait partie d'un domaine de protéine de faisceau à trois hélices.

5. Polypeptide de liaison au PDGF-Rβ selon l'une quelconque des revendications précédentes, qui comprend une séquence d'acides aminés choisie parmi :
i) K-[*PBM*]-DPSQSXₐX_{b}LLX_{c}EAKKLNDX_{d}Q ;
dans laquelle
[*PBM*] représente un motif de liaison au PDGF-Rβ tel que défini dans l'une quelconque des revendications 1 à 3 ;
Xₐ est choisi parmi A et S ;
X_{b} est choisi parmi N et E ;
X_{c} est choisi parmi A et S ;
X_{d} est choisi parmi A et S ;
et
ii) une séquence d'acides aminés qui possède une identité d'au moins 80 % à l'une quelconque des séquences définies ci-dessus.

6. Polypeptide de liaison au PDGF-Rβ selon la revendication 5, dans lequel i) est choisi parmi les SEQ ID NO: 180-358.

7. Polypeptide de liaison au PDGF-Rβ selon la revendication 6, dans lequel i) est choisi parmi les SEQ ID NO: 181-182, la SEQ ID NO: 184, la SEQ ID NO: 186, la SEQ ID NO: 190, la SEQ ID NO: 239, la SEQ ID NO: 251, la SEQ ID NO: 257, la SEQ ID NO: 290, la SEQ ID NO: 332 et la SEQ ID NO: 358.

8. Polypeptide de liaison au PDGF-Rβ selon l'une quelconque des revendications précédentes, dont la séquence d'acides aminés comprend une séquence choisie parmi les SEQ ID NO: 359-537.

9. Polypeptide de liaison au PDGF-Rβ selon la revendication 8, dont la séquence d'acides aminés comprend une séquence choisie parmi les SEQ ID NO: 360-361, la SEQ ID NO: 363, la SEQ ID NO: 365, la SEQ ID NO: 369, la SEQ ID NO: 418, la SEQ ID NO: 430, la SEQ ID NO: 436, la SEQ ID NO: 469, la SEQ ID NO: 511 et la SEQ ID NO: 537.

10. Polynucléotide codant pour un polypeptide selon l'une quelconque des revendications précédentes.

11. Polypeptide de liaison au PDGF-Rβ selon l'une quelconque des revendications 1 à 9, à utiliser comme médicament.

12. Combinaison d'un polypeptide de liaison au PDGF-Rβ selon l'une quelconque des revendications 1 à 9 et d'un agent thérapeutique.

13. Combinaison selon la revendication 12, à utiliser comme médicament.

14. Polypeptide de liaison au PDGF-Rβ ou combinaison selon l'une quelconque des revendications 1 à 9 et 12, pour le traitement d'une affection liée au PDGF-Rβ choisie parmi une maladie cancéreuse telle que les gliomes, les sarcomes et les leucémies; des troubles vasculaires tels que l'athérosclérose, la resténose, l'hypertension pulmonaire et des maladies rétiniennes ; des maladies fibrotiques telles que la fibrose pulmonaire, la cirrhose du foie, la sclérodermie, la glomérulosclérose et la fibrose cardiaque.
